# EUROPEAN PATENT APPLICATION

(11) **EP 4 785 853 A1**
(43) Date of publication of application: **05.08.2026**
(21) Application number: 24872263.9
(22) Date of filing: 25.09.2024
(51) Int. Cl.: A61B 3/10

(54) **OPTICAL COHERENCE TOMOGRAPHY DEVICE, CONTROL METHOD THEREFOR, AND PROGRAM**

(30) Priority: 26.09.2023 JP 2023163739
(71) Applicant: Topcon Corporation, Tokyo 174-8580 (JP)
(72) Inventor: ISHIKAWA, Akiko, Tokyo 174-8580 (JP); MORIGUCHI, Yoshikiyo, Tokyo 174-8580 (JP); MINO, Toshihiro, Tokyo 174-8580 (JP); TAMURA, Masato, Tokyo 174-8580 (JP); KUJI, Riku, Tokyo 174-8580 (JP)
(74) Representative: Gagel, Roland
(86) International application number: PCT/JP2024/034149
(87) International publication number: WO 2025/070477

(57) **Abstract**

An optical coherence tomography apparatus includes an optical system, a storage unit, and a controller. The optical system is configured to allow an optical condition to be changed, and to acquire optical coherence tomography data of an object to be measured by scanning the object to be measured with measurement light. The storage unit stores in advance setting information for each scan region in the object to be measured, the setting information being information for setting an optical condition of the optical system so that image quality of an image formed based on the optical coherence tomography data becomes equal to or greater than a predetermined image quality level. The controller is configured to change an optical condition of the optical system based on the setting information, and to perform optical coherence tomography on the object to be measured, in accordance with the scan region of the measurement light in the object to be measured.

## Description

### [TECHNICAL FIELD]

This invention relates to an optical coherence tomography apparatus, a method of controlling the optical coherence tomography apparatus, and a program.

### [BACKGROUND ART]

Optical coherence tomography (OCT) apparatuses that are used to form images representing the surface morphology or the internal morphology of an object to be measured using light beam emitted from a laser light source or the like have been known. OCT performed in the OCT apparatuses is not invasive on the human body, and therefore is expected to be applied to the medical field or the biological field, in particular. For example, in the ophthalmic field, apparatuses for forming images of the fundus, the cornea, or the like have been in practical use. Such apparatuses using a method of OCT (OCT apparatuses) can be applied to observe various sites of an eye to be examined. In addition, because of the ability to acquire high-definition images, the OCT apparatuses are applied to the diagnosis of various eye diseases.

In case of imaging (photographing) the object to be measured at a wide angle, optimal optical conditions of an optical system for imaging differ for each scan region, depending on a cross-sectional shape of the object to be measured. As a result, image quality (measurement accuracy) differs for each scan region. Therefore, the image quality around the site of interest may not be sufficient, and the object to be measured may need to be re-imaged.

For example, Patent Document 1 discloses a method of acquiring two-dimensional images in a predetermined imaging range of an eye to be examined by synthesizing a plurality of two-dimensional images acquired by performing scan on different positions in the predetermined imaging range of the eye to be examined with thinning.

### [PRIOR ART DOCUMENTS]

### [PATENT DOCUMENTS]

[Patent Document 1] Japanese Unexamined Patent Publication No. 2022-69735

### [SUMMARY OF THE INVENTION]

### [PROBLEMS TO BE SOLVED BY THE INVENTION]

However, even if the method disclosed in Patent Document 1 is used, re-imaging, that is necessary due to the different image quality in each scan region, cannot be avoided. Thus, the time required for re-imaging increases, and the burden on the examinee increases when the object to be measured is a living eye.

The present invention has been made in view of such circumstances, and one of objects of the present invention is to provide a new technique for acquiring images of an object to be measured with good image quality while avoiding re-imaging as much as possible in case of imaging the object to be measured at a wide angle.

### [MEANS OF SOLVING THE PROBLEMS]

One aspect of some embodiments is an optical coherence tomography apparatus including: an optical system configured to allow an optical condition to be changed, and to acquire optical coherence tomography data of an object to be measured by scanning the object to be measured with measurement light; a storage unit that stores in advance setting information for each scan region in the object to be measured, the setting information being information for setting an optical condition of the optical system so that image quality of an image formed based on the optical coherence tomography data becomes equal to or greater than a predetermined image quality level; and a controller configured to change an optical condition of the optical system based on the setting information to perform optical coherence tomography on the object to be measured, in accordance with the scan region of the measurement light in the object to be measured.

Another aspect of the embodiments is a method of controlling an optical coherence tomography apparatus including an optical system configured to allow an optical condition to be changed, and to acquire optical coherence tomography data of an object to be measured by scanning the object to be measured with measurement light. The method of controlling the optical coherence tomography apparatus includes a control step of changing an optical condition of the optical system based on setting information, the setting information being information for setting an optical condition of the optical system so that image quality of an image formed based on the optical coherence tomography data becomes equal to or greater than a predetermined image quality level, and of performing optical coherence tomography on the object to be measured, for each scan region in the object to be measured, in accordance with the scan region of the measurement light in the object to be measured.

Still another aspect of the embodiments is a program of causing a computer to execute each step of method of controlling the optical coherence tomography apparatus described above.

Still another aspect of the embodiments is a computer program product including the computer program(s)/instruction(s). The computer program product realizes each step in the method of controlling the optical coherence tomography apparatus described above, when the computer program(s)/instruction(s) is/are executed by the processor.

Still another aspect of the embodiments is a computer readable storage medium (recording medium) in which the computer program(s)/instruction(s) is/are stored. The computer readable recording medium realizes each step of the method of controlling the optical coherence tomography apparatus according to the embodiments, when the computer program(s)/instruction(s) is/are executed by the processor.

### [EFFECTS OF THE INVENTION]

According to embodiments according to the present invention, a new technique for acquiring images of an object to be measured with good image quality while avoiding re-imaging as much as possible in case of imaging the object to be measured at a wide angle can be provided.

### [BRIEF EXPLANATION OF THE DRAWINGS]

[FIG. 1] FIG. 1 is a schematic diagram illustrating an example of a configuration of an optical system of an ophthalmic apparatus according to a first embodiment.
[FIG. 2] FIG. 2 is a schematic diagram illustrating an example of a configuration of an optical system of the ophthalmic apparatus according to the first embodiment.
[FIG. 3] FIG. 3 is a schematic diagram illustrating an example of a configuration of an optical system of the ophthalmic apparatus according to the first embodiment.
[FIG. 4] FIG. 4 is a schematic diagram illustrating an example of a configuration of a processing system of the ophthalmic apparatus according to the first embodiment.
[FIG. 5] FIG. 5 is a schematic diagram illustrating an example of a configuration of a processing system of the ophthalmic apparatus according to the first embodiment.
[FIG. 6] FIG. 6 is an explanatory diagram of an operation of the ophthalmic apparatus according to the first embodiment.
[FIG. 7] FIG. 7 is an explanatory diagram of an operation of the ophthalmic apparatus according to the first embodiment.
[FIG. 8] FIG. 8 is a flowchart illustrating an example of an operation of the ophthalmic apparatus according to the first embodiment.
[FIG. 9] FIG. 9 is an explanatory diagram of an operation of the ophthalmic apparatus according to the first embodiment.
[FIG. 10] FIG. 10 is an explanatory diagram of an operation of the ophthalmic apparatus according to the first embodiment.
[FIG. 11] FIG. 11 is an explanatory diagram of an operation of the ophthalmic apparatus according to the first embodiment.
[FIG. 12] FIG. 12 is a flowchart illustrating an example of an operation of the ophthalmic apparatus according to the first embodiment.
[FIG. 13] FIG. 13 is a flowchart illustrating an example of an operation of the ophthalmic apparatus according to the first embodiment.
[FIG. 14] FIG. 14 is a schematic diagram illustrating an example of a configuration of an optical system of the ophthalmic apparatus according to a second embodiment.
[FIG. 15] FIG. 15 is a schematic diagram illustrating an example of a configuration of a processing system of the ophthalmic apparatus according to the second embodiment.
[FIG. 16] FIG. 16 is a schematic diagram illustrating an example of a configuration of a processing system of the ophthalmic apparatus according to a third embodiment.
[FIG. 17] FIG. 17 is a flowchart illustrating an example of an operation of the ophthalmic apparatus according to the third embodiment.
[FIG. 18] FIG. 18 is an explanatory diagram of an operation of the ophthalmic apparatus according to the third embodiment.

### [MODES FOR CARRYING OUT THE INVENTION]

Referring now to the drawings, exemplary embodiments of an optical coherence tomography apparatus, a method of controlling the optical coherence tomography apparatus, and a program according to the present invention are described below. Any of the contents of the documents cited in the present specification and arbitrary known techniques may be applied to the embodiments below.

An optical coherence tomography (OCT) apparatus according to the embodiments includes an optical system configured to allow an optical condition of the optical system to be changed. The optical system includes an interference optical system configured to acquire optical coherence tomography data of an object to be measured by scanning the object to be measured with measurement light. The OCT apparatus stores setting information for each of plurality of scan region in the object to be measured. Here, the setting information is information for setting the optical condition of the optical system described above so that image quality of an image formed based on the OCT data becomes equal to or greater than a predetermined image quality level. For example, the setting information is obtained by performing provisional measurement. For example, in the main measurement after the provisional measurement, the OCT apparatus changes an optical condition of the optical system based on the setting information stored in advance and performs OCT on the object to be measured, in accordance with the scan region of the measurement light in the object to be measured.

Examples of the optical condition of the optical system include a focusing position of the measurement light used for OCT, a polarization component of the measurement light, an aberration correction component, an optical path length difference between the measurement light and reference light, and a position of the optical system relative to the object to be measured.

For example, the optical system includes one or more optical elements, and is configured to allow any of the focusing position of the measurement light used for OCT, the polarization component of the measurement light, the aberration correction component, and the optical path length difference between the measurement light and the reference light to be changed by controlling the one or more optical elements.

For example, the optical system includes a mechanism that moves the optical system or the one or more optical elements, and is configured to allow any of the focusing position of the measurement light used for OCT, the polarization component of the measurement light, the aberration correction component, and the optical path length difference between the measurement light and the reference light to be changed by controlling the mechanism.

For example, the OCT apparatus includes a movement mechanism that moves the optical system relative to the object to be measured, and is configured to allow the position of the optical system relative to the object to be measured to be changed by controlling the movement mechanism.

The focusing position is changed by the focusing position changing member as an optical element. Examples of the focusing position changing member include a lens that is movable along an optical axis, a liquid crystal lens, and an Alvarez lens.

The polarization component is changed by a polarization component changing member as an optical element. Examples of the polarization component include a polarization controller. In case of splitting light from a light source into the measurement light and the reference light, that are used for OCT, the polarization controller may change the polarization component of the light from the light source, or the polarization component of the measurement light.

The aberration correction component is changed by an aberration correction device as an optical element. Examples of the aberration correction device include a variable cross cylinder (hereinafter referred to as VCC) lens, a liquid crystal lens, a wavefront aberration correction optical system including a deformable mirror, and an Alvarez lens.

The optical path length difference is changed by an optical path length changing member as an optical element or a mechanism. Examples of the optical path length changing member include a member that changes at least one of the optical path length of the measurement light or the optical path length of the reference light.

The position of the optical system relative to the object to be measured is changed by a mechanism that changes at least one of the object to be measured or the optical system. Examples of the mechanism include a movement mechanism that changes a relative position between the object to be measured and a holding member that holds the optical system.

Examples of the image quality equal to or greater than a predetermined image quality level include image quality in which the evaluation value of image quality for the entire image is maximized, image quality in which the evaluation value of image quality for the entire image is equal to or greater than a predetermined threshold value level, image quality in which a statistical value of the evaluation values, each of which is calculated for each scan region, of a plurality of images is maximized, and image quality in which a statistical value of the evaluation values, each of which is calculated for each scan region, of a plurality of images is equal to or greater than a predetermined threshold value level. Here, examples of the statistical value include a maximum value, a minimum value, a median, an average value, a mode, a range, a variance, a standard deviation, a weighted average value where a weighting factor is larger the closer to the site of interest (optical axis of the optical system), or a value of a predetermined evaluation equation using any of the statistical values described above.

Such setting information of the optical condition of the optical system is identified, for example, in the provisional measurement (provisional imaging (photographing, shooting), provisional scan) performed before the main measurement (main imaging, main scan). In the main measurement, OCT is performed on the object to be measured using the optical system whose optical condition has been changed based on the setting information identified in the provisional measurement.

Thereby, the image of the object to be measured can be acquired with good image quality regardless of the cross-sectional shape of the object to be measured without repeating the main measurement multiple times, and the measurement time (imaging time) can be shortened. In particular, in case that the object to be measured is a living eye, the burden on the examinee can be reduced. In some embodiments, the image quality can be checked (estimated) before performing the main measurement.

In some embodiments, the scan region includes a central region including an optical axis of the optical system in the object be measured, and one or more peripheral regions around the central region. For example, the one or more peripheral regions includes one or more intermediate regions that are arranged outside the central region and surround the central region, and the peripheral region that is arranged outside the outermost intermediate region among the one or more intermediate regions and surrounds the outermost intermediate region.

Thereby, the image of the object to be measured can be acquired with good image quality, even when the inclination of the cross-section differs between the central region and the one or more peripheral regions of the object to be measured.

A method of controlling the OCT apparatus according to the embodiments is a method for controlling the OCT apparatus according to the embodiments. A method of controlling the OCT apparatus according to the embodiments includes one or more steps performed by the OCT apparatus described above. A program (computer program)/instruction according to the embodiments causes a computer to execute each step in the method of controlling the OCT apparatus according to the embodiments. A computer program product according to the embodiments includes the computer program(s)/instruction(s). The computer program product realizes each step of the method of controlling the OCT apparatus according to the embodiments, when the computer program(s)/instruction(s) are executed by the processor. A recording medium (storage medium) according to the embodiments is any computer readable non-transitory recording medium (storage medium) on which the program according to the embodiments is recorded. The computer readable recording medium according to the embodiments stores the computer program(s)/instruction(s). The computer readable recording medium realizes each step of the method of controlling the OCT apparatus according to the embodiments, when the computer program(s)/instruction(s) are executed by the processor. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like. Further, the program may be transmitted and received through a network such as the Internet, LAN, etc.

Hereinafter, an ophthalmic apparatus in which the object to be measured is a living eye will be described as an example of the OCT apparatus according to the embodiments. However, the embodiments can also be applied to OCT apparatus where the object to be measured is other than the living eye.

The ophthalmic apparatus according to the embodiments can perform OCT on an arbitrary site of the eye to be examined, such as a fundus, or an anterior segment, for example. In this specification, an image acquired using OCT may be collectively referred to as an "OCT image". Further, the measurement operation for forming OCT images may be referred to as OCT measurement (main measurement, provisional measurement).

Hereinafter, in the embodiments, the case of using the swept source type OCT method in the measurement and the imaging (photographing) using OCT will be described. However, the configuration according to the embodiments can also be applied to an ophthalmic apparatus using other type of OCT (for example, spectral domain type OCT or time domain OCT).

Furthermore, hereinafter, a case in which the evaluation value of image quality is higher the better the image quality is, and is lower the worse the image quality is, will be described. However, the embodiments can be applied to a case in which the evaluation value of image quality is lower the better the image quality is, and is higher the worse the image quality is. In this case, in the following explanation, "high evaluation value" should be read as "low evaluation value", "evaluation value equal to or greater than a threshold value" should be read as "evaluation value equal to or less than a threshold value", and "evaluation value less than a threshold value" should be read as "evaluation value greater than a threshold value".

### <First embodiment>

### [Configuration]

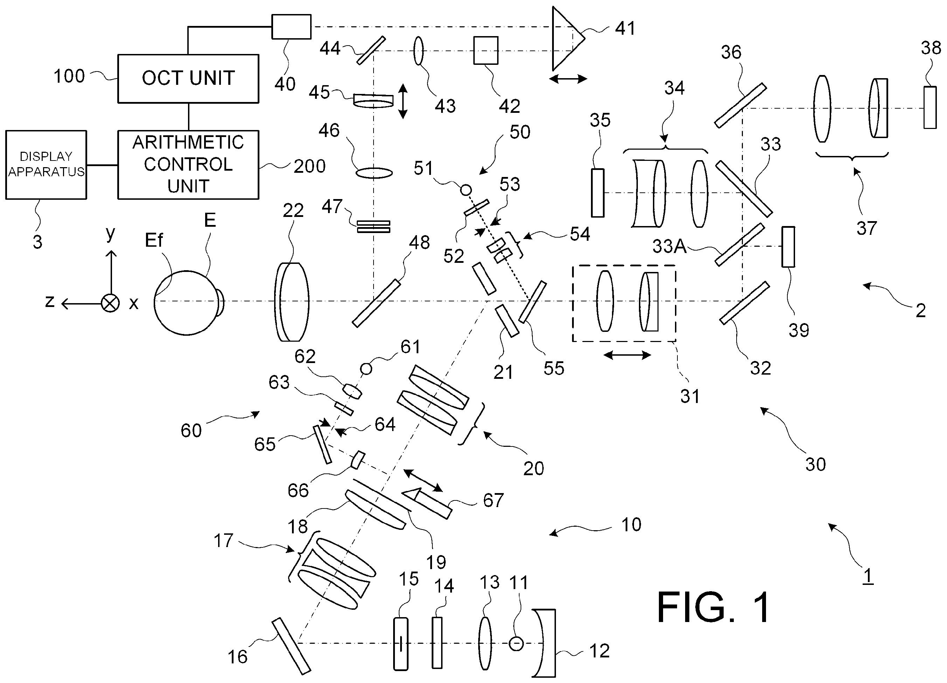

As shown in FIG. 1, FIG. 2, and FIG. 3, an ophthalmic apparatus 1 according to a first embodiment includes a fundus camera unit 2, an OCT unit 100, and an arithmetic control unit 200. The fundus camera unit 2 has substantially the same optical system as the conventional fundus camera. The OCT unit 100 is provided with an optical system for obtaining OCT images (for example, tomographic images) of the fundus (or the anterior segment). The arithmetic control unit 200 is provided a computer(s) that executes various kinds of arithmetic processing, control processing, and the like.

### [Fundus camera unit 2]

The fundus camera unit 2 illustrated in FIG. 1 is provided with an optical system for acquiring two-dimensional images (fundus images) representing the surface morphology of a fundus Ef of an eye E to be examined. Examples of the fundus images include observation images and photographic images. The observation image is, for example, a monochrome moving image formed at a predetermined frame rate using near-infrared light. The photographic image may be, for example, a color image captured by flashing visible light, or a monochrome still image using near-infrared light or visible light as illumination light. The fundus camera unit 2 may be configured to be capable of acquiring other types of images such as fluorescein angiograms, indocyanine green angiograms, and autofluorescent angiograms.

The fundus camera unit 2 is provided with a jaw holder and a forehead rest for supporting the face of a subject (examinee). Further, the fundus camera unit 2 is provided with an illumination optical system 10 and an imaging optical system 30. The illumination optical system 10 irradiates illumination light onto the fundus Ef. The imaging optical system 30 guides fundus reflected light of this illumination light to an imaging device (i.e., the CCD image sensor 35 or 38). Each of the CCD image sensors 35 and 38 is sometimes simply referred to as a "CCD". Further, the imaging optical system 30 guides measurement light coming from the OCT unit 100 to the fundus Ef, and guides the measurement light via the fundus Ef to the OCT unit 100.

An observation light source 11 in the illumination optical system 10 includes, for example, a halogen lamp. Light (observation illumination light) emitted from the observation light source 11 is reflected by a reflective mirror 12 having a curved reflective surface, travels through a condenser lens 13, and becomes near-infrared light after passing through a visible cut filter 14. Further, the observation illumination light is once converged near an imaging light source 15, is reflected by a mirror 16, and passes through relay lenses 17 and 18, a diaphragm 19, and a relay lens 20. Then, the observation illumination light is reflected on the peripheral part (the surrounding area of the hole part) of the perforated mirror 21, is transmitted through a dichroic mirror 48, and refracted by the objective lens 22, thereby illuminating the fundus Ef. It should be noted that an LED (light emitting diode) may be used as the observation light source.

Fundus reflected light of the observation illumination light is refracted by the objective lens 22, is transmitted through the dichroic mirror 48, passes through the hole part formed in the center area of the perforated mirror 21, is transmitted through a dichroic mirror 55, travels through a focusing lens 31, and is reflected by a mirror 32. Further, this fundus reflected light is transmitted through a half mirror 33A, is reflected by a dichroic mirror 33, and forms an image on the light receiving surface of the CCD image sensor 35 by a condenser lens 34. The CCD image sensor 35 detects the fundus reflected light at a predetermined frame rate, for example. An image (observation image) based on the fundus reflected light detected by the CCD image sensor 35 is displayed on a display apparatus 3. It should be noted that when the imaging optical system 30 is focused on the anterior segment, an observation image of the anterior segment of the eye E to be examined is displayed.

The imaging light source 15 includes, for example, a xenon lamp. Light (imaging illumination light) emitted from the imaging light source 15 is irradiated onto the fundus Ef through the same route as that of the observation illumination light. The fundus reflected light of the imaging illumination light is guided to the dichroic mirror 33 via the same route as that of the observation illumination light, is transmitted through the dichroic mirror 33, is reflected by a mirror 36, and forms an image on the light receiving surface of the CCD image sensor 38 by a condenser lens 37. The display apparatus 3 displays an image (photographic image) obtained based on the fundus reflected light detected by the CCD image sensor 38. It should be noted that the display apparatus 3 for displaying the observation image and the display apparatus 3 for displaying the photographic image may be the same or different. Besides, when similar imaging is performed by illuminating the eye E to be examined with infrared light, an infrared photographic image is displayed. It is also possible to use an LED as the imaging light source.

A liquid crystal display (LCD) 39 displays a fixation target and a visual target used for visual acuity measurement. The fixation target is a visual target for fixating the eye E to be examined, and is used when performing fundus imaging (photography) and OCT measurement.

Part of light emitted from the LCD 39 is reflected by the half mirror 33A, is reflected by the mirror 32, travels through the focusing lens 31 and the dichroic mirror 55, and passes through the hole part of the perforated mirror 21. The light having passed through the hole part of the perforated mirror 21 is transmitted through the dichroic mirror 48, and is refracted by the objective lens 22, thereby being projected onto the fundus Ef.

By changing the display position of the fixation target on the screen of the LCD 39, the fixation position of the eye E to be examined can be changed. Examples of the fixation position of the eye E to be examined include a position for acquiring an image centered at a macular region of the fundus Ef, a position for acquiring an image centered at an optic disc, and a position for acquiring an image centered at the fundus center between the macular region and the optic disc. Further, the display position of the fixation target may be changed to any desired position.

In addition, as with a conventional fundus camera, the fundus camera unit 2 is provided with an alignment optical system 50 and a focus optical system 60. The alignment optical system 50 generates an indicator (referred to as an alignment indicator) for the position matching (i.e., the alignment) of the optical system with respect to the eye E to be examined. The focus optical system 60 generates a target (split indicator) for adjusting the focus with respect to the fundus Ef.

The light output from an LED 51 of the alignment optical system 50 (i.e., alignment light) travels through the diaphragms 52 and 53 and the relay lens 54, is reflected by the dichroic mirror 55, and passes through the hole part of the perforated mirror 21. The light having passed through the hole part of the perforated mirror 21 is transmitted through the dichroic mirror 48, and is projected onto the cornea of the eye E to be examined by the objective lens 22.

Cornea reflected light of the alignment light travels through the objective lens 22, the dichroic mirror 48 and the hole part described above. Part of the cornea reflected light is transmitted through the dichroic mirror 55, and passes through the imaging focusing lens 31, is reflected by the mirror 32, and is transmitted through the half mirror 33A. The cornea reflected light transmitted through the half mirror 33A is reflected by the dichroic mirror 33, and forms an image on the light receiving surface of the CCD image sensor 35 by the condenser lens 34. The light receiving image (i.e., alignment indicator image) captured by the CCD image sensor 35 is displayed on the display apparatus 3 together with the observation image. A user performs an alignment in the same manner as performed on a conventional fundus camera. Alternatively, alignment may be performed in such a way that the arithmetic control unit 200 analyzes the position of the alignment indicator and moves the optical system (automatic alignment).

To perform focus adjustment, a reflective surface of a reflection rod 67 is arranged in a slanted position on an optical path of the illumination optical system 10. The light output from a LED 61 in the focus optical system 60 (i.e., focus light) passes through a relay lens 62, is split into two light beams by a split indicator plate 63, passes through a two-hole diaphragm 64, and is reflected by a mirror 65. The focus light reflected by the mirror 65 is once converged on the reflective surface of the reflection rod 67 by the condenser lens 66, and is reflected by the reflective surface. Further, the focus light travels through the relay lens 20, is reflected by the perforated mirror 21, is transmitted through the dichroic mirror 48, and is refracted by the objective lens 22, thereby being projected onto the fundus Ef.

Fundus reflected light of the focus light passes through the same route as the cornea reflected light of the alignment light and is detected by the CCD image sensor 35. The display apparatus 3 displays the light receiving image (split indicator) captured by the CCD image sensor 35 together with the observation image. As in the conventional case, the arithmetic control unit 200 analyzes the position of the split indicator, and moves the focusing lens 31 and the focus optical system 60 for focusing (automatic focusing). Alternatively, the user may manually perform the focus adjustment while visually checking the split indicator.

The dichroic mirror 48 branches the optical path for OCT measurement from the optical path for fundus imaging (photography). The dichroic mirror 48 reflects light of wavelengths used for OCT measurement, and transmits light for fundus imaging. The optical path for OCT measurement is provided with, in order from the OCT unit 100 side, a collimator lens unit 40, an optical path length changing unit 41, an optical scanner 42, a collimator lens 43, a mirror 44, an OCT focusing lens 45. a field lens 46, and a VCC lens 47.

The optical path length changing unit 41 is configured to be capable of moving in a direction indicated by the arrow in FIG. 1, thereby changing the optical path length for OCT measurement. The change in the optical path length is used for the correction of the optical path length according to the axial length of the eye E to be examined, and/or for the adjustment of the interference state, or the like. The optical path length changing unit 41 includes, for example, a corner cube and a mechanism for moving the corner cube.

The optical scanner 42 is disposed at a position conjugate optically to a pupil of the eye E to be examined (pupil conjugate position) or near the position. The optical scanner 42 changes the traveling direction of light (measurement light) traveling along the optical path for OCT measurement. The optical scanner 42 can deflect the measurement light in a one-dimensionally or two-dimensional manner, under the control from the arithmetic control unit 200 described below.

The optical scanner 42 includes a first galvanometer mirror, a second galvanometer mirror, and a mechanism for driving them independently, for example. The first galvanometer mirror deflects measurement light LS so as to scan the imaging site (fundus Ef or the anterior segment) in a horizontal direction (x-direction) orthogonal to an optical axis of the interference optical system. The second galvanometer mirror deflects the measurement light LS deflected by the first galvanometer mirror so as to scan the imaging site in a vertical direction (y direction) orthogonal to the optical axis of the interference optical system. Thereby, the imaging site can be scanned with the measurement light LS in any direction on the x-y plane.

For example, by controlling an orientation of the first galvanometer mirror and an orientation of the second galvanometer mirror included in the optical scanner 42 at the same time, the irradiated position of the measurement light can be moved along an arbitrary trajectory on the x-y plane. This allows to scan the imaging site according to a desired scan pattern.

The OCT focusing lens 45 is movable along the optical path of the measurement light LS (the optical axis of the interference optical system). The OCT focusing lens 45 moves along the optical path of the measurement light LS, under the control from the arithmetic control unit 200 described below.

In some embodiments, a liquid crystal lens or an Alvarez lens is provided instead of the OCT focusing lens 45. The liquid crystal lens or the Alvarez lens, as well as the OCT focusing lens 45, is controlled by the arithmetic control unit 200.

The VCC lens 47 is arranged on the optical path of the measurement light and changes at least one of the cylindrical power (astigmatic power) or the cylindrical axis angle (astigmatic axis angle). The VCC lens 47 has two cylindrical lenses (optical elements) positioned opposite each other, and is configured to change at least one of the cylindrical power or the cylindrical axis angle by changing at least one of the axial directions of the two cylindrical lenses. In the first embodiment, the two cylindrical lenses are configured to be capable of rotating independently so that the two axial directions are changed relative to each other. Further, the two cylindrical lenses are configured to be capable of rotating integrally while keeping the angle formed by the two axial directions.

The VCC lens 47 is disposed at a position conjugate optically to the pupil of the eye to be examined (pupil conjugate position) or near the position. In the first embodiment, since the optical scanner 42 is disposed at the position conjugate optically to the pupil of the eye to be examined, the VCC lens 47 is disposed near the position conjugate optically to the pupil of the eye to be examined.

For the purpose of correcting the astigmatic power of the eye E to be examined, even if the VCC lens 47 is disposed near the pupil conjugate position, it is reasonable to assume that a deviation of the arranged position of the VCC lens 47 relative to the pupil conjugate position has little effect on the cylindrical power or the cylindrical axis angle which are changed by the VCC lens 47.

When the VCC lens 47 is controlled based on the optometric data (objective measurement values or subjective inspection values) of the eye E to be examined, the optometric data are mainly measured values on the fovea of the eye E to be examined. However, it is reasonable to assume that the deviation of the arranged position of the VCC lens 47 relative to the pupil conjugate position has little effect on the cylindrical power or the cylindrical axis angle which are changed by the VCC lens 47. Therefore, even if the imaging site is different from the fovea, the VCC lens 47 may be positioned near the pupil conjugate position.

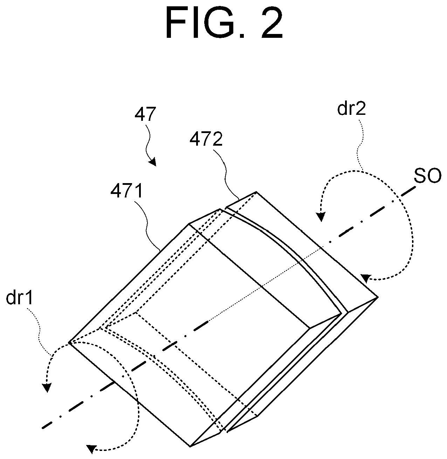

Such a VCC lens 47 is configured to include cylindrical lenses 471 and 472 with equal power and different signs from each other (focal lengths f0 and - f0), as shown in FIG. 2, for example. The cylindrical lens 471 (VCC1) has a convex surface (positive power), and is provided to be capable of rotating in a rotational direction dr1 around the optical path of measurement light LS (optical axis SO of the interference optical system). The cylindrical lens 472 (VCC2) has a concave surface (negative power), and is provided to be capable of rotating in a rotational direction dr2 around the optical axis SO. The cylindrical lenses 471 and 472 are driven by a driver such as a pulse motor, and are rotated independently around the optical axis SO. When the cylindrical lenses 471 and 472 are rotated in opposite directions to each other, the cylindrical power is changed, and when the cylindrical lenses 471 and 472 are rotated integrally in the same direction, the cylindrical axis angle is changed.

For example, the cylindrical lenses 471 and 472 are rotated in opposite directions to each other from a state in which the cylindrical axis angle of the cylindrical lenses 471 and 472 are tilted at a predetermined angle (e.g., 45 degrees) with respect to the optical axis SO. Thereby, arbitrary cylindrical power can be generated. Further, by rotating the cylindrical lenses 471 and 472 integrally in the same direction, arbitrary cylindrical axis angle can be generated.

### [OCT unit 100]

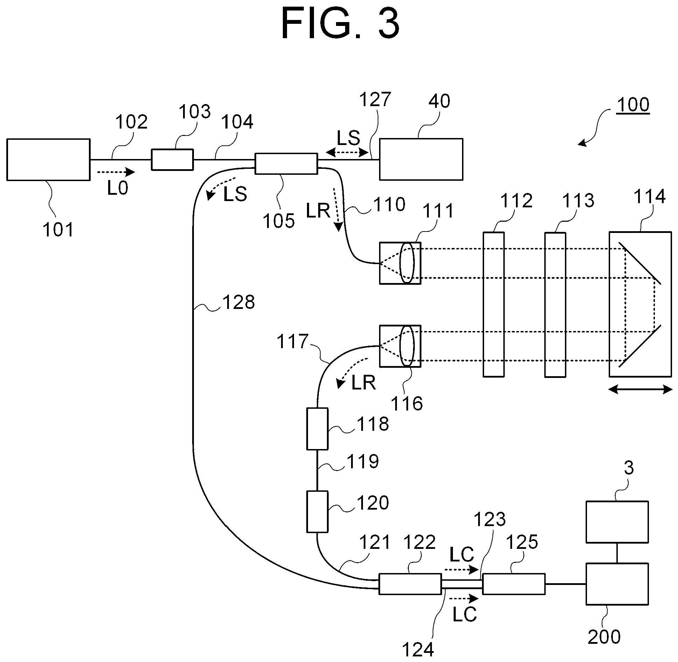

The configuration of the OCT unit 100 will be described with reference to FIG. 3. The OCT unit 100 is provided with an optical system for acquiring OCT images of the fundus Ef. The optical system has the same configuration as the conventional swept source type OCT apparatus. That is, the optical system includes an interference optical system configured to split light from a wavelength scanning type (wavelength sweeping type) light source into measurement light and reference light, to make the measurement light returned from the fundus Ef and the reference light having passed through a reference optical path interfere with each other to generate interference light, and to detect the interference light. The detection result (detection signal) of the interference light obtained by the interference optical system is a signal indicating the spectra of the interference light and is sent to the arithmetic control unit 200.

Like the general swept source type OCT apparatus, a light source unit 101 includes a wavelength scanning type (wavelength sweeping type) light source capable of scanning (sweeping) the wavelengths of emitted light. The light source unit 101 temporally changes the output wavelengths within the near-infrared wavelength bands that cannot be visually recognized with human eyes.

The light L0 emitted from the light source unit 101 is guided to a polarization controller 103 through an optical fiber 102, and a polarization state of the light L0 is adjusted. The polarization controller 103, for example, applies external stress to the looped optical fiber 102 to thereby adjust the polarization state of the light L0 guided through the optical fiber 102.

The light L0 whose polarization state has been adjusted by the polarization controller 103 is guided to a fiber coupler 105 through an optical fiber 104, and is split into the measurement light LS and the reference light LR.

The reference light LR is guided to the collimator 111 through the optical fiber 110 and becomes a parallel light beam. The reference light LR, which has become the parallel light beam, is guided to a corner cube 114 via an optical path length correction member 112 and a dispersion compensation member 113. The optical path length correction member 112 acts as a delay means for matching the optical path length (i.e., the optical distance) of the reference light LR and that of the measurement light LS. The dispersion compensation member 113 acts as a dispersion compensation means for matching the dispersion characteristic of the reference light LR and that of the measurement light LS.

The corner cube 114 reverses the traveling direction of the reference light LR that has become the parallel light beam by the collimator 111. The optical path of the reference light LR incident on the corner cube 114 and the optical path of the reference light LR emitted from the corner cube 114 are parallel to each other. Further, the corner cube 114 is movable in a direction along the incident light path and the emitting light path of the reference light LR. Through such movement, the optical path length of the reference light LR (i.e., the reference optical path) is varied.

The reference light LR that has traveled through the corner cube 114 passes through the dispersion compensation member 113 and the optical path length correction member 112, is converted from the parallel light beam to a convergent light beam by a collimator 116, and enters an optical fiber 117. The reference light LR that has entered the optical fiber 117 is guided to a polarization controller 118. With the polarization controller 118, the polarization state of the reference light LR is adjusted.

The polarization controller 118 has the same configuration as, for example, the polarization controller 103. The reference light LR whose polarization state has been adjusted by the polarization controller 118 is guided to an attenuator 120 through an optical fiber 119, and the light quantity of the reference light LR is adjusted under the control of the arithmetic control unit 200. The reference light LR whose light quantity has been adjusted by the attenuator 120 is guided to the fiber coupler 122 through the optical fiber 121.

The measurement light LS generated by the fiber coupler 105 is guided through an optical fiber 127 and is collimated into a parallel light beam by the collimator lens unit 40. The measurement light LS made into a parallel light beam reaches the dichroic mirror 48 via the optical path length changing unit 41, the optical scanner 42, the collimator lens 43, the mirror 44, the OCT focusing lens 45, the field lens 46, and the VCC lens 47. Subsequently, the measurement light LS is reflected by the dichroic mirror 48, is refracted by the objective lens 22, and is projected onto the fundus Ef. The measurement light LS is scattered and reflected (including reflection) at various depth positions of the fundus Ef. Back-scattered light of the measurement light LS from the fundus Ef reversely advances along the same path as the outward path, and is guided to the fiber coupler 105. Then, the back-scattered light passes through an optical fiber 128, and arrives at the fiber coupler 122.

The fiber coupler 122 combines (interferes) the measurement light LS incident through the optical fiber 128 and the reference light LR incident through the optical fiber 121 to generate interference light. The fiber coupler 122 generates a pair of interference light LC by splitting the interference light generated from the measurement light LS and the reference light LR at a predetermined splitting ratio (for example, 50 : 50). The pair of the interference light LC emitted from the fiber coupler 122 is guided to the detector 125 through the optical fibers 123 and 124, respectively.

The detector 125 is, for example, a balanced photodiode that includes a pair of photodetectors for respectively detecting the pair of interference light LC and outputs the difference between the pair of detection results obtained by the pair of photodetectors. The detector 125 sends the detection result (i.e., detection signal) to the arithmetic control unit 200. For example, the arithmetic control unit 200 performs the Fourier transform etc. on the spectral distribution based on the detection result obtained by the detector 125 for each series of wavelength scanning (i.e., for each A-line) to form the tomographic image as the OCT image. The arithmetic control unit 200 causes the formed image to be displayed on the display apparatus 3.

Although a Michelson interferometer is employed in the first embodiment, it is possible to employ any type of interferometer such as Mach-Zehnder-type as appropriate. In the present embodiment, in addition to the configuration shown in FIG. 3, the interference optical system may further include the collimator lens unit 40, the optical path length changing unit 41, the optical scanner 42, the collimator lens 43, the mirror 44, the OCT focusing lens 45, the field lens 46, and the VCC lens 47, which are shown in FIG. 1. This interference optical system is an example of the "interference optical system" according to the embodiments. The VCC lens 47 (and VCC driver 47A described below) is an example of the "astigmatism correction optical member" according to the embodiments. The OCT focusing lens 45 (and OCT focusing driver 45A described below) is an example of the "focusing position changing member" according to the embodiments. At least one of the optical path length changing unit 41 or the corner cube 114 (and reference driver 114A described below) is an example of the "optical path length changing member" according to the embodiments. At least one of the polarization controller 103 or 118 is an example of the "polarization state changing member" according to the embodiments.

### [Arithmetic control unit 200]

The configuration of the arithmetic control unit 200 will be described.

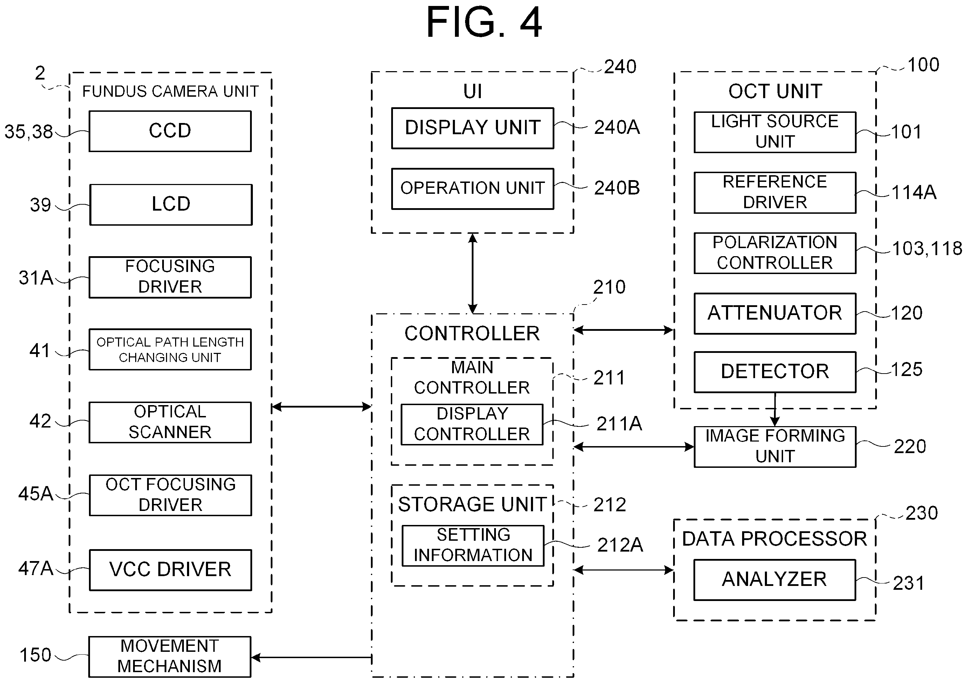

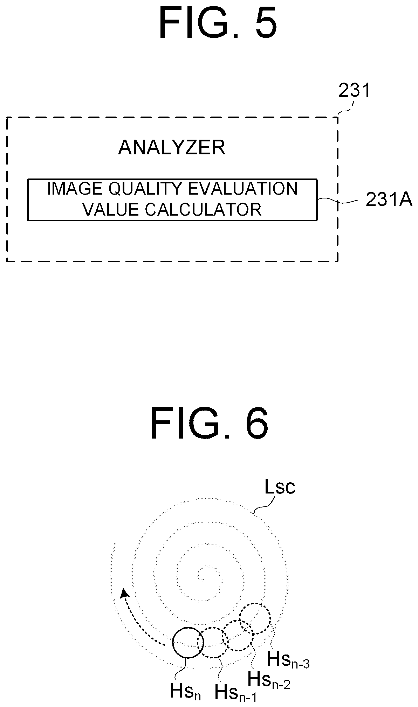

FIG. 4, and FIG. 5 show block diagrams of examples of a configuration of a processing system (control system) of the ophthalmic apparatus 1 according to the first embodiment. FIG. 5 shows a functional block diagram representing an example of a configuration of an analyzer 231 in FIG. 4. In FIG. 4, like reference numerals designate like parts as in FIG. 1, FIG. 2, and FIG. 3. The same description may not be repeated below.

The arithmetic control unit 200 analyzes the detection signals fed from the detector 125 to form an OCT image of the fundus Ef (or anterior segment). The arithmetic processing therefor is performed in the same manner as in the conventional swept-source-type OCT apparatus.

As shown in FIG. 4, the arithmetic control unit 200 includes a controller 210, and controls each part of the fundus camera unit 2, the display apparatus 3, and the OCT unit 100. For example, the arithmetic control unit 200 forms an OCT image, and causes the formed OCT image to be displayed on the display apparatus 3.

Examples of the control for the fundus camera unit 2 include the operation control for the observation light source 11, the imaging light source 15, the LEDs 51 and 61, the operation control for the CCD image sensors 35 and 38, the operation control for the LCD 39, the movement control for the focusing lens 31, the movement control for the OCT focusing lens 45, the movement control for the reflection rod 67, the operation control for the alignment optical system 50, the movement control for the focus optical system 60, the movement control for the optical path length changing unit 41, the driving control for the VCC lens 47, and the operation control for the optical scanner 42.

Examples of the control for the OCT unit 100 include the operation control for the light source unit 101, the movement control for the corner cube 114, the operation control for the detector 125, the operation control for the attenuator 120, and the operation controls for the polarization controllers 103 and 118.

Like conventional computers, the arithmetic control unit 200 includes a microprocessor, a RAM (random access memory), a ROM (read only memory), a hard disk drive, a communication interface, and the like. A storage device such as the hard disk drive stores a computer program for controlling the ophthalmic apparatus 1. The arithmetic control unit 200 may include various kinds of circuitry such as a circuit board for forming OCT images. In addition, the arithmetic control unit 200 may include an operation device (or an input device) such as a keyboard and a mouse, and a display device such as an LCD.

The processor includes, for example, a circuit(s) such as, for example, a CPU (central processing unit), a GPU (graphics processing unit), an ASIC (application specific integrated circuit), and a PLD (programmable logic device). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program. At least a part of the storage circuit or the storage apparatus may be included in the processor. Further, at least a part of the storage circuit or the storage apparatus may be provided outside of the processor. In some embodiments, the functions of the arithmetic control unit 200 are realized by one or more processors.

The fundus camera unit 2, the display apparatus 3, the OCT unit 100, and the arithmetic control unit 200 may be integrally provided (i.e., in a single housing), or they may be separately provided in two or more housings.

The controller 210 includes a main controller 211 and a storage unit 212.

### (Main controller 211)

The main controller 211 performs various controls by outputting control signals to each part of the ophthalmic apparatus 1 described above. In particular, the main controller 211 controls the CCD image sensors 35 and 38, the LCD 39, the focusing driver 31A, the optical path length changing unit 41, the optical scanner 42, the OCT focusing driver 45A, and the VCC driver 47A for the fundus camera unit 2. Further, the main controller 211 controls the light source unit 101, the reference driver 114A, the polarization controllers 103 and 118, the attenuator 120, and the detector 125 for the OCT unit 100.

The main controller 211 controls an exposure time (charge accumulation time), a sensitivity, a frame rate, or the like of the CCD image sensor 35 or 38. In some embodiments, the main controller 211 controls the CCD image sensor 35 or 38 so as to acquire images having the desired image quality.

The main controller 211 performs display control of fixation targets or visual targets for the visual acuity measurement, for the LCD 39. Thereby, the visual target presented to the eye E to be examined can be switched, or type of the visual targets can be changed. Further, the presentation position of the visual target to the eye E to be examined can be changed by changing the display position of the visual target on the screen of the LCD 39.

The focusing driver 31A moves the focusing lens 31 in the optical axis direction. The main controller 211 controls the focusing driver 31A so that the focusing lens 31 is positioned at a desired focusing position. As a result, the focusing position of the imaging optical system 30 is changed.

For example, the main controller 211 analyzes the position of the split indicator in the light receiving image (split indicator) obtained by the CCD image sensor 35, and controls the focusing driver 31A and the focus optical system 60. Alternatively, for example, the main controller 211 controls the focusing driver 31A and the focus optical system 60, according to the operations performed by the user on the operation unit 240B described below, while displaying a live image of the eye E to be examined on the display unit 240A described below.

The main controller 211 controls the optical path length changing unit 41 to change the optical path length of the measurement light LS. Thereby, the difference between the optical path length of the measurement light LS and the optical path length of the reference light LR is changed.

For example, the main controller 211 analyzes the detection result of the interference light LC obtained by OCT measurement (or the OCT image formed based on the detection result), and controls the optical path length changing unit 41 so that the measurement site is positioned at a desired depth position.

The main controller 211 is configured to control the optical scanner 42. The main controller 211 controls the optical scanner 42 so as to deflect the measurement light LS according to the deflection pattern corresponding to the scan mode set in advance.

Examples of scan mode like this include a line scan, a cross scan, a circle scan, a radial scan, a concentric scan, a multiline cross scan, a spiral scan, a Lissajous scan, a three-dimensional scan, and an ammonite scan.

The line scan is a scan mode in which the measurement light LS is deflected so that the movement trajectory of the irradiated positions of the measurement light LS at the imaging site (measurement site) is line-shaped. The line direction of the movement trajectory can be changed (rotatable) around the optical axis on the x-y plane.

For example, the line scan includes a horizontal scan and a vertical scan. The horizontal scan is a scan mode in which measurement light LS is deflected so that the movement trajectory of the irradiated positions of the measurement light LS is in the horizontal direction (x-direction). The horizontal scan also includes a mode in which the measurement light LS is deflected along a plurality of scan lines that are arranged in the vertical direction (y-direction) and extend in the horizontal direction. In this mode, the intervals between the scan lines may be set as desired. Further, the intervals between the scan lines may be set sufficiently small to form a three-dimensional image. Such a scan mode is referred to as three-dimensional scan. These items for the horizontal scan mode may be applied to the vertical scan mode in similar ways.

The cross scan is a scan mode in which the measurement light LS is deflected so that the movement trajectory of the irradiated positions of the measurement light LS at the imaging site (measurement site) is cross-shaped. For example, the cross scan can be performed by executing two line scans whose line directions cross each other. The angle at which the two line scans intersect can be changed. In some embodiments, the scan lengths in the B-scan direction of the two line scans are identical. In some embodiments, the scan lengths in the B-scan direction of the two line scans are different.

The circle scan is a scan mode in which the measurement light LS is deflected so that the movement trajectory of the irradiated positions of the measurement light LS at the imaging site (measurement site) is, for example, in a circle around the optical axis SO. For example, in the circle scan, the measurement light LS is deflected so that the movement trajectory is a perfect circle, ellipse, or arc (part of a circumference).

The radial scan is a scan mode in which the measurement light LS is deflected so that the movement trajectory of the irradiated positions of the measurement light LS at the imaging site (measurement site) is radial around the optical axis SO, for example. In the radial scan mode, the irradiated positions of the measurement light LS are moved along a radial trajectory consisting of a plurality of straight lines arranged via a predetermined angle. The cross scan described above is one mode of the radial scan.

For example, in the radial scan, two or more line scans with B-scan directions different from each other are performed. In some embodiments, the scan lengths in the B-scan direction of the two or more line scans are identical. In some embodiments, the scan length in the B-scan direction of at least one of the two or more line scans is different from the other scan lengths.

The concentric scan is a scan mode in which the measurement light LS is deflected so that the movement trajectory of the irradiated positions of the measurement light LS at the imaging site (measured site) is concentric around the optical axis SO, for example. For example, in the concentric scan, the measurement light LS is deflected so that the movement trajectory of each circle is a perfect circle, ellipse, or arc (part of a circumference). In the concentric circle according to some embodiments, a plurality of circle scans with different diameters are performed in combination with each other. The circle scan is one mode of the concentric scan.

The multiline cross scan is a scan pattern in which a group of horizontal scan lines (for example, 5 lines) parallel to each other and a group of vertical scan lines (for example, 5 lines) parallel to each other are arranged so as to be perpendicular to each other in the vicinity of the center positions of the both groups of scan lines.

For example, in each group of scan lines in the multiline cross scan, two or more line scans are performed. In some embodiments, the scan lengths in the B-scan direction of the two or more line scans are identical. In some embodiments, the scan length in the B-scan direction of at least one of the two or more line scans is different from the other scan lengths.

The spiral scan (helical scan) is a scan mode in which the measurement light LS is deflected so that the movement trajectory of the irradiated positions of the measurement light LS at the imaging site (measurement site) is spiral around the optical axis SO, for example. In the spiral scan mode, the irradiated positions of the measurement light LS are moved along a spiral trajectory while the rotation radius is gradually reduced (or increased).

The Lissajous scan is a scan mode in which the measurement light LS is deflected so that the movement trajectory of the irradiated positions of the measurement light LS at the imaging site (measurement site) follows the Lissajous curve. The Lissajous scan is disclosed in Japanese Unexamined Patent Application Publication No. 2018-68578, for example.

The ammonite scan is one of scan modes in which a slow scan (low-speed scan) and a fast scan (high-speed scan) are combined. Specifically, the ammonite scan is a scan mode in which a scan reference position (scan center position) of the circle scan as a fast scan is moved along the scan pattern of the spiral scan as a slow scan. In other words, the circle scans are sequentially performed around each scan center position while moving the scan center position along the spiral scan pattern.

By scanning the imaging site with the measurement light LS according to the deflection pattern corresponding to the scan mode as described above, the tomographic image as the OCT image in the plane stretched by the direction along the scan line (scan trajectory) and the fundus depth direction (z-direction) can be acquired.

The OCT focusing driver 45A moves the OCT focusing lens 45 along the optical axis of the measurement light LS. The main controller 211 controls the OCT focusing driver 45A so that the OCT focusing lens 45 is positioned at a desired focusing position. As a result, the focusing position of the measurement light LS is changed. The focusing position of the measurement light LS corresponds to the depth position (z position) of the beam waist of the measurement light LS.

For example, the main controller 211 controls the OCT focusing driver 45A based on a signal-to-noise ratio of the detection result of the interference light LC obtained by OCT measurement or evaluation value(s) (including statistical value of the evaluation values) corresponding to the image quality of the OCT image formed based on the detection result.

When the liquid crystal lens or the Alvarez lens is provided in place of the OCT focusing lens 45, the main controller 211 can control the liquid crystal lens or the Alvarez lens in the same way as it controls the OCT focusing driver 45A.

The VCC driver 47A rotates the cylindrical lenses 471 and 472 independently each other around the optical axis of the measurement light LS. Thereby, at least one of the cylindrical power or the cylindrical axis angle is changed.

For example, the main controller 211 controls the VCC driver 47A based on a signal-to-noise ratio of the detection result of the interference light LC obtained by OCT measurement or evaluation value(s) (including statistical value of the evaluation values) corresponding to the image quality of the OCT image formed based on the detection result. In some measurement light, the main controller 211 controls the VCC driver 47A based on a signal-to-noise ratio of the detection result of the interference light LC obtained by deflecting the measurement light LS according to the deflection pattern corresponding to the circle scan or evaluation value(s) (including statistical value of the evaluation values) corresponding to the image quality of the OCT image formed based on the detection result.

When a liquid crystal lens, a deformable mirror, or an Alvarez lens is provided in place of the VCC lens 47, the main controller 211 can control the liquid crystal lens, the deformable mirror, or the Alvarez lens in the same way as it controls the VCC driver 47A.

The main controller 211 controls the light source unit 101. The control for the light source unit 101 includes switching the light source on and off, controlling the intensity of the emitted light, changing the center frequency of the emitted light, changing the sweep speed of the emitted light, changing the sweep frequency, and changing the sweep wavelength range.

The reference driver 114A moves the corner cube 114 provided on the optical path of the reference light along this optical path. Thereby, the difference between the optical path length of the measurement light LS and the optical path length of the reference light LR is changed.

For example, the main controller 211 analyzes the detection result of the interference light LC obtained by OCT measurement (or the OCT image formed based on the detection result), and controls the reference driver 114A so that the measurement site is positioned at a desired depth position. In some embodiments, any one of the optical path length changing unit 41 and the reference driver 114A is provided.

The main controller 211 controls the polarization controllers 103 and 118. For example, the main controller 211 controls the polarization controllers 103 and 118 based on a signal-to-noise ratio of the detection result of the interference light LC obtained by OCT measurement or evaluation value(s) (including statistical value of the evaluation values) corresponding to the image quality of the OCT image formed based on the detection result.

The main controller 211 controls the attenuator 120. For example, the main controller 211 controls the attenuator 120 based on a signal-to-noise ratio of the detection result of the interference light LC obtained by OCT measurement or evaluation value(s) (including statistical value of the evaluation values) corresponding to the image quality of the OCT image formed based on the detection result.

The main controller 211 controls the detector 125. The control for the detector 125 includes the control for an exposure time (charge accumulation time), a sensitivity, a frame rate, or the like of the detector 125.

The movement mechanism 150 relatively moves the fundus camera unit 2 (OCT unit 100) relative to the eye E to be examined three-dimensionally. For example, the main controller 211 is capable of controlling the movement mechanism 150 to three-dimensionally move the optical system installed in the fundus camera unit 2. This control is used for alignment and/or tracking. Here, the tracking is to move the optical system of the apparatus according to the movement of the eye E to be examined. To perform tracking, alignment and focusing are performed in advance. The tracking is performed by moving the optical system of the apparatus in real time according to the position and orientation of the eye E to be examined based on the image obtained by photographing moving images of the eye E to be examined, thereby maintaining a suitable positional relationship in which alignment and focusing are adjusted.

In some embodiments, the main controller 211 corrects the position of scan range for OCT imaging in real time, based on tracking information obtained by performing tracking (tracking information obtained by tracking the optical system (interference optical system) with respect to a movement of the eye E to be examined). The main controller 211 can control the optical scanner 42 so as to scan the corrected scan range with the measurement light LS.

In some embodiments, the main controller 211 controls the optical system constituting the ophthalmic apparatus 1 based on the setting information stored in advance in the storage unit 212 to change the optical condition(s) (imaging condition, measurement condition) of the optical system.

In the present embodiment, the provisional measurement (provisional imaging) is performed before the main measurement (main imaging). By changing the optical condition(s) of the optical system based on the detection result of the interference light LC acquired in the provisional measurement or the OCT image formed from the detection result, the measurement conditions for the main measurement are adjusted.

As shown in FIG. 4, the main controller 211 includes a display controller 211A.

The display controller 211A causes the various information to be displayed on the display apparatus 3 (or display unit 240A described below). Examples of the information displayed on the display apparatus 3 include the results of imaging (observation image, OCT image (image of the eye to be examined formed based on the detection result of the interference light LC obtained by scanning with the measurement light LS), the measurement result (measured value), the evaluation value of the image quality of the image described below, the statistical value calculated based on the evaluation values, and evaluation metric information corresponding to the evaluation value. The display controller 211A can cause the evaluation value of the image quality of the OCT image, the statistical value, or the evaluation metric information to be displayed on the display apparatus 3 in association with the OCT image.

Further, the main controller 211 performs a process of writing data in the storage unit 212 and a process of reading out data from the storage unit 212.

### (Storage unit 212)

The storage unit 212 stores various types of data. Examples of the data stored in the storage unit 212 include image data of an OCT image, image data of a fundus image, and information on the eye to be examined. The information on the eye to be examined includes information on the examinee such as patient ID and name, and information on the eye to be examined such as identification information of the left/right eye. In addition, the storage unit 212 stores the optometric data obtained by an external device (e.g., a refractometer or subjective inspection apparatus) in advance, and various programs and data for operating the ophthalmic apparatus 1. The optometric data includes an astigmatic power of the eye to be examined, and an astigmatic axis angle of the eye to be examined. The optometric data may further include a spherical power of the eye to be examined. The optometric data may include at least one of a spherical power of the eye to be examined, an astigmatic power of the eye to be examined, or an astigmatic axis angle of the eye to be examined.

Further, the storage unit 212 stores setting information 212A. The setting information 212A is information for setting the optical condition(s) of the optical system constituting the ophthalmic apparatus 1. More specifically, the setting information 212A is information for setting the optical condition(s) of the optical system so that image quality of an image formed based on the OCT data, which is acquired using the optical system constituting the ophthalmic apparatus 1, becomes equal to or greater (higher) than a predetermined image quality level. Examples of the optical condition of the optical system constituting the ophthalmic apparatus 1 include a focusing position of the measurement light LS used for OCT, a polarization component of the measurement light LS, an aberration correction component, an optical path length difference between the measurement light LS and reference light LR, and a position of the optical system relative to the eye E to be examined.

The main controller 211 can control the CCD image sensors 35 and 38, the LCD 39, the focusing driver 31A, the optical path length changing unit 41, the optical scanner 42, the OCT focusing driver 45A, the VCC driver 47A, the light source unit 101, the reference driver 114A, the polarization controllers 103 and 118, the attenuator 120, the detector 125, and the movement mechanism 150, based on the setting information 212A.

At least part of the above data stored in the storage unit 212 may be stored in a storage unit provided outside the ophthalmic apparatus 1. For example, the ophthalmic apparatus 1 is connected so as to be capable of communicating with a sever apparatus having a function of storing at least part of the above data via a network such as an in-hospital LAN (Local Area Network). Here, the ophthalmic apparatus 1 and the server apparatus are connected via a WAN (Wide Area Network) such as the Internet. Further, the ophthalmic apparatus 1 and the server apparatus may be connected via a network that combines the LAN and the WAN.

### (Image forming unit 220)

An image forming unit 220 forms image data of the OCT image of the fundus Ef or the anterior segment based on the detection signal (interference signal, OCT data) from the detector 125. That is, the image forming unit 220 forms the image data of the eye E to be examined based on the detection result of the interference light LC obtained by the interference optical system. This processing includes processes such as noise removal (noise reduction), filter processing, and fast Fourier transform (FFT) in the same manner as the conventional swept source type OCT. The image data acquired in this manner is a data set including a group of image data formed by imaging the reflection intensity profiles of a plurality of A-lines. Here, the A-lines are the paths of the measurement light LS in the eye E to be examined.

In order to improve the image quality, it is possible to repeatedly perform scan with the same pattern a plurality of times to acquire a plurality of data sets, and to compose (i.e., average) the plurality of data sets.

The image forming unit 220 includes, for example, the circuitry described above. It should be noted that "image data" and an "image" based on the image data may not be distinguished from each other in the present specification. In addition, a site of the fundus Ef and an image of the site may not be distinguished from each other.

### (Data processor 230)

A data processor 230 performs various kinds of data processing (e.g., image processing) and various kinds of analysis processing on the detection result of the interference light LC or the image formed by the image forming unit 220. For example, the data processor 230 performs various correction processing, such as analysis processing of the signal-to-noise ratio of the interference signal, image brightness correction, dispersion correction, etc.

Further, the data processor 230 performs various kinds of image processing and various kinds of analysis processing on images (fundus images, anterior segment images, etc.) obtained by the fundus camera unit 2.

The data processor 230 performs known image processing such as interpolation processing for interpolating pixels between two-dimensional tomographic images to form image data of the three-dimensional image of the fundus Ef or the eye E to be examined. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. When displaying an image based on volume data, the data processor 230 performs rendering processing (volume rendering, maximum intensity projection (MIP), etc.) on the volume data, thereby forming image data of a pseudo three-dimensional image viewed from a particular visual line. The pseudo three-dimensional image is displayed on the display device such as the display unit 240A.

Further, stack data of a plurality of tomographic images may be formed as the image data of the three-dimensional image. The stack data is image data obtained by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 230 can build (form) the OCTA image (blood vessel emphasized image, angiogram) in which retinal blood vessels and choroidal blood vessels are emphasized (highlighted), based on data (for example, B-scan data, OCT data) acquired in time series by performing OCT scan. The OCTA image may be an image representing a cross-sectional structure or a front image.

In some embodiments, the data processor 230 compares the two OCT images acquired by repeatedly performing B-scans on the approximately same site of the eye E to be examined, and converts the pixel value of a change portion of the signal intensity into a pixel value corresponding to a change amount in the change portion to build the emphasized image (motion contrast image) in which the change portion is emphasized. Further, the data processor 230 can form the OCTA (angiography) image by extracting information of a predetermined thickness amount at a desired site from a plurality of built emphasized images and building as an en-face image.

The data processor 230 can perform position matching between the fundus image and the OCT image. When the fundus image and the OCT image are obtained in parallel, the position matching between the fundus image and the OCT image, which have been (almost) simultaneously obtained, can be performed using the optical axis of the imaging optical system 30 as a reference. Such position matching can be achieved since the optical system for the fundus image and that for the OCT image are coaxial. Besides, regardless of the timing of obtaining the fundus image and the OCT image, position matching between the fundus image and the OCT image can be achieved by registering the fundus image with an image obtained by projecting the OCT image onto the x-y plane. This position matching method can also be employed when the optical system for obtaining the fundus image and the optical system for OCT measurement are not coaxial. Further, when both the optical systems are not coaxial, if the relative positional relationship between these optical systems is known, the position matching can be performed with referring to the relative positional relationship in a manner similar to the case of coaxial optical systems.

### (Analyzer 231)

The data processor 230 includes an analyzer 231. In addition to the analysis processing described above, the analyzer 231 can perform calculation processing of the evaluation value (including the statistical value of the evaluation values) corresponding to the image quality (for example, signal-to-noise ratio) of the image. In the calculation processing of the evaluation value, the evaluation value is calculated by analyzing the detection result of the interference light, the OCT image, or a segmented image. Here, the OCT is formed by the image forming unit 220. The segmented image is obtained by segmenting (dividing) the OCT image. The segmented image may be an image obtained by segmenting the OCT image, corresponding to each of a plurality of scan regions obtained by segmenting the scan region. Alternatively, the segmented image may be an image obtained by segmenting the OCT image, corresponding to each of a preset plurality of scan regions.

The main controller 211 identifies the optical condition(s) of the optical system described above, in which the image quality is equal to or greater than a predetermined image quality level, for the entire OCT image or for each segmented image corresponding to the scan region based on the analysis result obtained by the analyzer 231, and to identify the setting information for setting the identified optical condition(s). The main controller 211 can identify the setting information for each segmented image corresponding to each of the plurality of scan regions.

The analyzer 231 includes an image quality evaluation value calculator 231A, as shown in FIG. 5.

The image quality evaluation value calculator 231A calculates arbitrary evaluation values quantitatively representing the image quality. Typically, the evaluation value is calculated so that the higher the quality of the image (image quality) is, the larger the value is. The calculation processing of the evaluation value performed by image quality evaluation value calculator 231A may be arbitrary processing. For example, the image quality evaluation value calculator 231A can perform processing using any known technology, such as the signal-to-noise ratio (SNR), the contrast-to-noise ratio (CNR), the root mean square (RMS) granularity, the Wiener spectrum, the modulation transfer function (MTF), the quality index

### (QI).

In the first example of the calculation processing of the evaluation value, the image quality evaluation value calculator 231A calculates a differential histogram of the image to be calculated, and calculates the image quality evaluation value using the maximum value of luminance values (differential values) with the number of pixels equal to or greater than a predetermined percentage in the entire image in the calculated differential histogram. Specifically, the image quality evaluation value calculator 231A calculates a difference between the maximum value of the differential values and the minimum value of the differential values at a predetermined threshold value (e.g., 20%) or higher as the evaluation value of the image quality (image quality evaluation value). In this case, the image quality evaluation value indicates a high value when the image quality is equal to or greater than a predetermined image quality level, and decreases as the image quality deviates from the predetermined image quality level.

In the second example of the calculation processing of the evaluation value, the data processor 230 (or analyzer 231) applies a predetermined analysis processing (e.g., segmentation processing) to an evaluation region set for an image corresponding to a predetermined site in the eye E to be examined. Thereby, the image quality evaluation value calculator 231A identifies an image regions (signal regions) corresponding to a desired site (tissue) and other image regions (non-signal regions). Next, the image quality evaluation value calculator 231A generates a histogram of luminance in the signal regions and a histogram of luminance in the non-signal regions. Subsequently, the image quality evaluation value calculator 231A calculates the evaluation value corresponding to the image quality from the degree of overlap of these two histograms. For example, the evaluation value is defined in the range of 0 to 100, such that when both histograms completely overlap, the evaluation value = 0, and when both histograms are completely separated, the evaluation value = 100. This evaluation operation may include, for example, normalization of two histograms, generation of a probability distribution function, and calculation of the evaluation value using a predetermined arithmetic formula.

Alternatively, the image quality evaluation value calculator 231A may calculate known parameters expressing the image quality, such as signal-to-noise ratio (SNR), spatial resolution, and contrast, for the image to be calculated. In this case, the image quality evaluation value indicates a high value when the image quality is equal to or greater than a predetermined image quality level, and decreases as the image quality deviates from the predetermined image quality level.

The image quality evaluation value calculator 231A calculates the evaluation value of the image quality of the entire OCT image, or the evaluation value of the image quality for each segmented image corresponding to each of the plurality of scan regions.

Further, the image quality evaluation value calculator 231A can calculate the statistical value of the evaluation values for a plurality of segmented images. Examples of the statistical value include a maximum value, a minimum value, a median, an average value, a mode, a range, a variance, a standard deviation, a weighted average value where a weighting factor is larger the closer to the site of interest (optical axis of the optical system), or a value of a predetermined evaluation equation using any of the statistical values described above.

Examples of the evaluation equation include an equation such that the higher the total value of the evaluation values of the segmented images and the smaller the variation of the evaluation values of the segmented images are, the larger the value VCCQ of the evaluation equation is. In this case, for example, the value VCCQ of the evaluation equation is a value obtained by dividing the sum of the evaluation values of the plurality of segmented images by (standard deviation of the plurality of evaluation values + 1).

In the present embodiment, the main controller 211 identifies the setting information for setting the optical condition(s) of the optical system described above based on the evaluation value of the image quality calculated by such the image quality evaluation value calculator 231A or the statistical value of the evaluation values calculated for each segmented image. For example, the main controller 211 identifies the optical condition(s) of the optical system described above, in which the image quality is equal to or greater than a predetermined image quality level, based on the evaluation value of the image quality of the entire image, and identifies the setting information for setting the identified optical condition(s). For example, the main controller 211 identifies the optical condition(s) of the optical system described above, in which the image quality is equal to or greater than a predetermined image quality level, based on the statistical value of the evaluation values of the image for each segmented image corresponding to each of the scan regions, and identifies the setting information for setting the identified optical condition(s).

For example, the main controller 211 identifies the setting information for setting the optical condition(s) of the optical system so that the evaluation value of the image quality or the statistical value of the evaluation values, which is calculated by the image quality evaluation value calculator 231A, becomes maximum value (minimum value or desired value), by repeating the change of the optical condition(s) of the optical system described above and the evaluation of the evaluation value of the image quality or the statistical value (evaluation equation) of the evaluation values, which is calculated by the image quality evaluation value calculator 231A.

The display controller 211A causes the evaluation value or the statistical value, which is calculated by the image quality evaluation value calculator 231A, to be displayed on the display apparatus 3 or display unit 240A (display means). In some embodiments, the display controller 211A causes the evaluation value of the segmented image to be displayed on the display apparatus 3, etc. associated with the segmented image, for at least one of the plurality of segmented images obtained by segmenting the OCT image.

The data processor 230 that functions described above includes, for example, a processor described above, a RAM, a ROM, a hard disk drive, a circuit board, and the like. Computer programs that cause a processor to execute the above functions are previously stored in a storage device such as a hard disk drive.

### (User interface 240)

The user interface 240 includes the display unit 240A and the operation unit 240B. The display unit 240A includes the display device of the arithmetic control unit 200 described above and/or the display apparatus 3. The operation unit 240B includes the operation device of the arithmetic control unit 200 described above. The operation unit 240B may include various kinds of buttons and keys provided on the housing of the ophthalmic apparatus 1, or provided outside the ophthalmic apparatus 1. For example, when the fundus camera unit 2 has a case similar to that of the conventional fundus camera, the operation unit 240B may include a joy stick, an operation panel, and the like provided to the case. Further, the display unit 240A may include various kinds of display devices, such as a touch panel placed on the housing of the fundus camera unit 2.

It should be noted that the display unit 240A and the operation unit 240B need not necessarily be formed as separate devices. For example, a device like a touch panel, which has a display function integrated with an operation function, can be used. In such cases, the operation unit 240B includes the touch panel and a computer program. The content of operation performed on the operation unit 240B is fed to the controller 210 as an electric signal. Moreover, operations and inputs of information may be performed using a graphical user interface (GUI) displayed on the display unit 240A and the operation unit 240B.

The eye E to be examined (fundus Ef, anterior segment) is an example of the "object to be measured" according to the embodiments. The optical system included in the OCT unit 100, the optical path length changing unit 41, the OCT focusing lens 45, and the VCC lens 47 (interference optical system) are an example of the "optical system" according to the embodiments. The OCT focusing lens 45, the corner cube 114, the optical path length changing unit 41, or the polarization controller 103 is an example of the "one or more optical elements" according to the embodiments. The focusing position of the measurement light LS, the polarization component of the measurement light LS, the aberration correction component, the optical path length difference between the measurement light LS and reference light LR, or the position of the optical system relative to the eye E to be examined is an example of the "optical condition of optical system" according to the embodiments. The ophthalmic apparatus 1 is an example of the "OCT apparatus" according to the embodiments. The optical path length changing unit 41, or, the corner cube 114 and reference driver 114A is an example of the "optical path length difference changing member" according to the embodiments. The OCT focusing lens 45 is an example of the "focusing lens" according to the embodiments. The VCC lens 47 is an example of the "aberration correction device (aberration correction member)" according to the embodiments. The display apparatus 3 or the display unit 240A is an example of the "display means" according to the embodiments.

Hereinafter, a case where the ophthalmic apparatus 1 according to the first embodiment performs ammonite scan as an OCT scan in the main measurement on the eye E to be examined will be described. However, the present embodiment also can be applied to cases where an OCT scan in the main measurement is performed on the eye E to be examined in a scan mode other than the ammonite scan.

FIG. 6 shows an explanatory diagram of the ammonite scan according to the first embodiment.

The ammonite scan according to the first embodiment is a scan mode in which one or more fast scans are sequentially performed with a scan pattern defined with reference to a moved scan center position, while the scan center position of the fast scan is moved along a scan pattern (scan trajectory) of the slow scan. In the ammonite scan according to the present embodiment, the fast scan is a circle scan and the slow scan is a spiral scan, where the circle scans, which are sequentially performed, are performed so that the circle scans intersect the scan pattern of the previous circle scan. In other words, the circle scan are sequentially performed so that the scan patterns of two adjacent circle scans intersect.

In FIG. 6, a scan pattern Lsc of the slow scan is schematically represented and fast scan patterns HSₙ (n is an integer of 4 or more), HSₙ₋₁, HSₙ₋₂, and HSₙ₋₃ are schematically represented.

The ammonite scan can perform scan on the eye E to be examined from a variety of scan directions with a simple control, by combining the slow scan and the fast scan, as described above. In particular, the region near the center of the ammonite scan (center position of slow scan) can be scanned at high density in a short period of time, and the farther away the scan position is from the center, the lower the density of the scan becomes in a longer period of time, which allows the scan of a wide region including the site of interest with a simple control.

According to the present embodiment, the eye E to be examined can be measured or imaged at a higher quality and wide angle without having to re-measuring or re-imaging, by scanning the eye E to be examined with the ammonite scan.

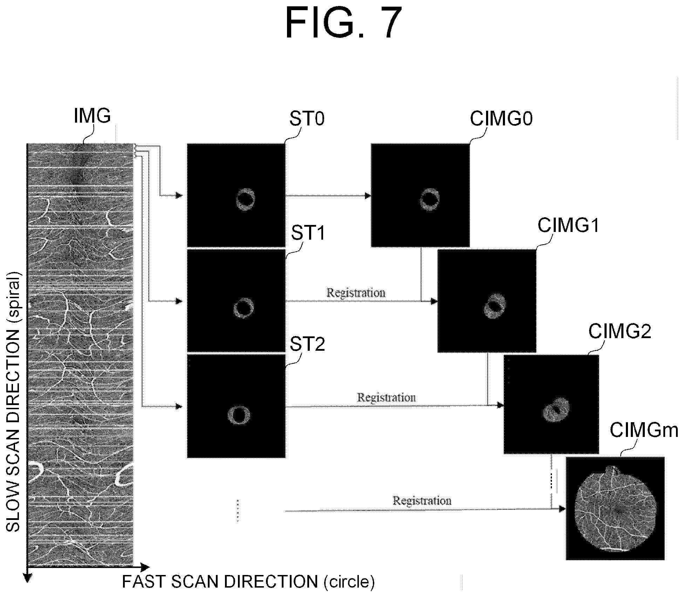

FIG. 7 schematically shows the OCT image formed based on the OCT data acquired by performing ammonite scan according to the first embodiment. In FIG. 7, a case where the OCTA image is formed will be described, for convenience of explanation. However, the same applies to the OCT image.

First, the main controller 211 sets the scan regions(s) of ammonite scan to the eye E to be examined. Examples of configuration parameter for setting the scan region of the ammonite scan include a scan start position of the slow scan (scan center position of slow scan), a scan end position of the slow scan, a scan speed of the slow scan, a change speed of scan radius from the scan center position of the slow scan, a scan speed of the fast scan, and a scan radius of the fast scan.

The main controller 211 control the optical scanner 42 and the OCT unit 100, etc. to repeat the movement of the scan center position of the fast scan along the scan pattern of the slow scan and two or more fast scans (circle scans) centered on the scan center positions after the movement. The main controller 211 controls the image forming unit 220 to form the OCT image (tomographic image, B-scan image) based on the acquired OCT data every time the circle scan is performed. Subsequently, the main controller 211 control the data processor 230 to sequentially form the OCTA images by comparing the two OCT images acquired by repeatedly performing circle scan (B-scan) on the approximately same site of the eye E to be examined. This allows to form the OCTA image IMG in which the OCTA images formed in the fast scan direction are arranged in the slow scan.

Next, the main controller 211 controls the data processor 230 to generate strip images ST0, ST1, ST2, ... by performing a coordinate transformation to the Cartesian coordinate system on each of a plurality of OCTA images formed in the fast scan direction. For example, the strip image ST0 is acquired by performing circle scan with the scan pattern HS₀ in FIG. 6. The strip image ST1 is acquired by performing circle scan with the scan pattern HS₁ intersecting the scan pattern HS₀. And, the strip image ST2 is acquired by performing circle scan with the scan pattern HS₂ intersecting the scan pattern HS₁. In this case, it is possible to thin out the OCTA images in which artifacts are generated due to the movement or blinking of the eye E to be examined during the circle scans, and to perform coordinate transformation merely on the OCTA images in which no artifacts are generated to acquire the strip images.

The main controller 211 controls the data processor 230 to generate synthetic images CIMG0, CIMG1, CIMG2, ..., CIMGm (m is an integer of 2 or more) by sequentially performing position matching (registration) of the strip images ST1, ST2, ..., using the strip image ST0 as the reference image. The data processor 230 performs position matching based on image (for example, vascular region in the OCTA image) within the scan region that overlaps in the two adjacent strip images.

Thereby, the synthetic image CIMGm in FIG. 7 can be acquired by performing ammonite scan in FIG. 6.

### [Operation Example]

The operation of the ophthalmic apparatus 1 according to the first embodiment will be described.

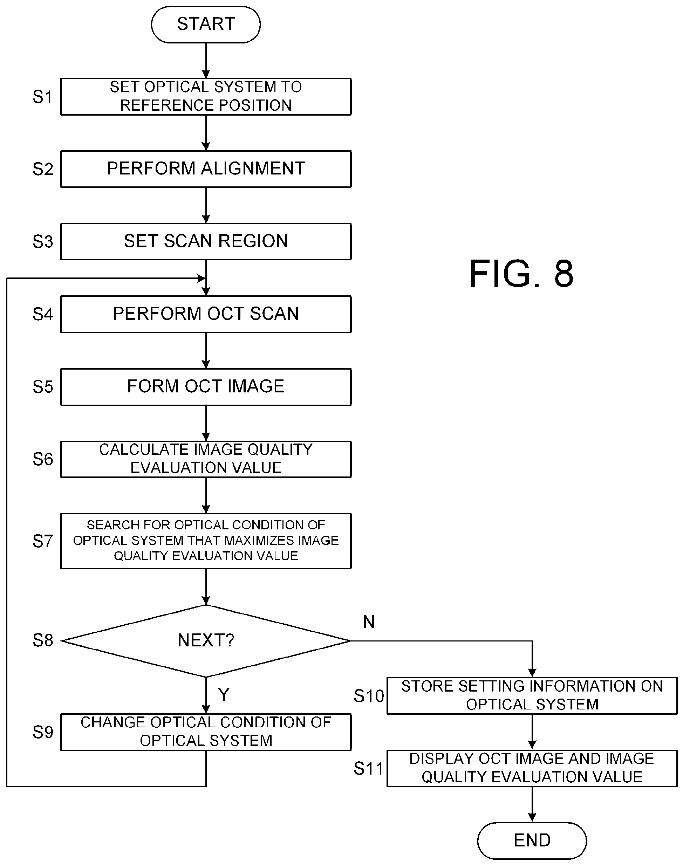

FIGs. 8 to 11 show explanatory diagrams of the operation in the provisional measurement performed before the main measurement in the ophthalmic apparatus 1 according to the first embodiment. For example, the circle scan is performed in the provisional measurement and the ammonite scan is performed in the main measurement. It should be noted that the ammonite scan may be performed in the provisional measurement. In addition, the circle scan may be performed in the main measurement.

FIG. 8 represents a flowchart of the example of the operation of the ophthalmic apparatus 1 according to the first embodiment. The storage unit 212 stores a computer program for realizing the processing shown in FIG. 8. The main controller 211 operates according to the computer program(s), and thereby the main controller 211 performs the processing shown in FIG. 8.

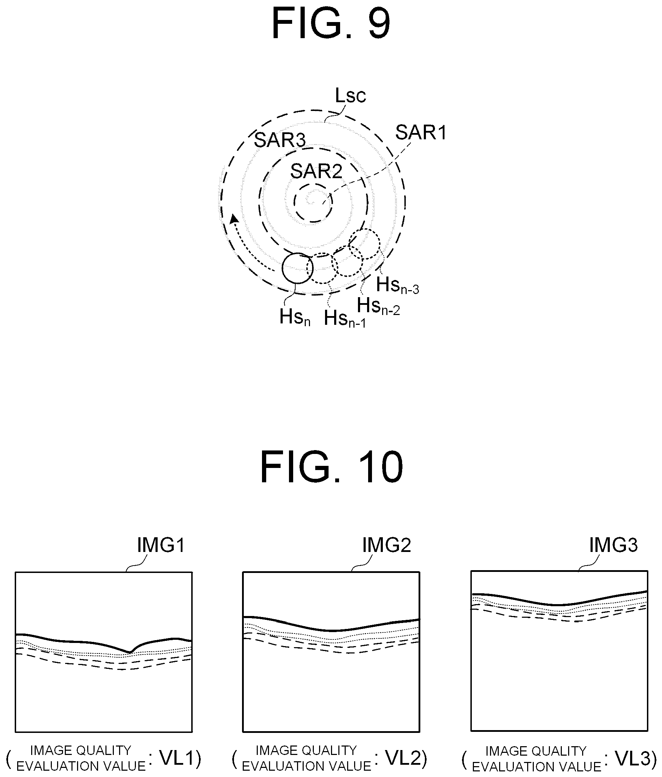

FIG. 9 schematically represents an example of a plurality of scan regions obtained by segmenting the scan region of the OCT scan in the provisional measurement performed in the ophthalmic apparatus 1 according to the first embodiment. In FIG. 9, the same parts as in FIG. 6 are shown by the same symbols and their descriptions will be omitted in an appropriate manner.

FIG. 10 schematically represents an example of a display of the OCT image and the image quality evaluation value which are obtained in the provisional measurement.

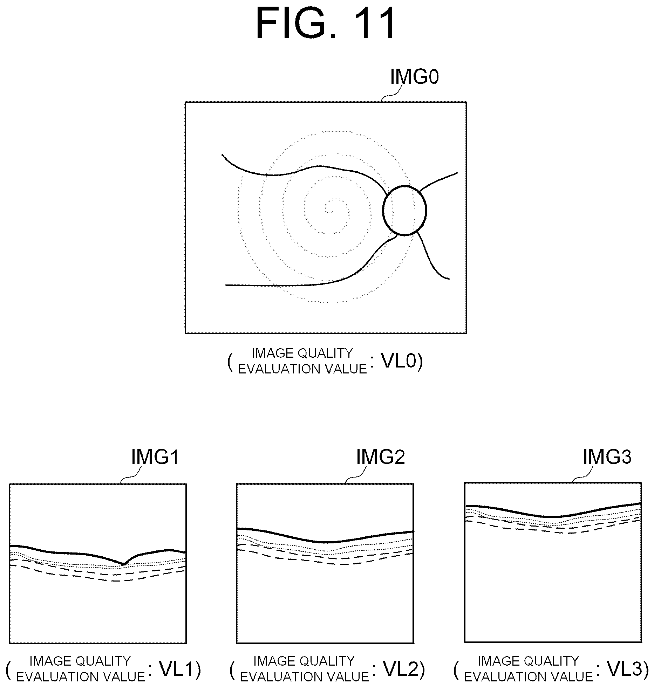

FIG. 11 schematically represents another example of a display of the OCT image and the image quality evaluation value which are obtained in the main measurement.

### (S1: Set optical system to reference position)

As shown in FIG. 8, first, the main controller 211 sets the optical conditions of the optical system constituting the ophthalmic apparatus 1 to a predetermined initial conditions, and sets the optical system to a reference position based on the set initial conditions.

### (S2: Perform alignment)

Subsequently, the main controller 211 performs alignment adjustment of the optical system relative to the eye E to be examined in a state where the fixation target is presented at a predetermined fixation position. Examples of the alignment adjustment include manual alignment and automatic alignment.

When the alignment adjustment is performed manually, the main controller 211 controls the alignment optical system 50 to project a pair of alignment indicators onto the eye E to be examined. A pair of alignment bright spots are displayed on the display unit 240A as the light receiving images of these alignment indicators. Further, the main controller 211 displays an alignment scale representing the target position of movement of the pair of alignment bright spots on the display unit 240A. The alignment scale is, for example, a bracket type image.

When the positional relationship between the eye E to be examined and the fundus camera unit 2 (objective lens 22) is appropriate, that is, when the distance (working distance) between the eye E to be examined and the fundus camera unit 2 is appropriate and the optical axis of the optical system of the fundus camera unit 2 and the ocular axis (corneal apex position) of the eye E to be examined are (approximately) coincident, the pair of alignment bright spots are formed at a predetermined position (for example, intermediate position between the corneal apex and the center of corneal curvature) respectively, and is projected onto the eye E to be examined, according to a known method. The examiner (user) can perform the alignment adjustment of the optical system to the eye E to be examined by moving the fundus camera unit 2 three-dimensionally so as to guide the pair of alignment bright spots into the alignment scale.

When the alignment adjustment is performed automatically, the movement mechanism 150 for moving the fundus camera unit 2 is used. The data processor 230 identifies the position of each alignment bright spot in the screen displayed on display unit 240A, and obtains a displacement between the identified position of each alignment bright point and the alignment scale. The main controller 211 controls the movement mechanism 150 to move the fundus camera unit 2 so as to cancel this displacement. Identifying the position of each alignment bright spot can be performed, for example, by obtaining the luminance distribution of each alignment bright spot and obtaining the position of the center of gravity based on this luminance distribution. Since the position of the alignment scale is constant, the desired displacement can be obtained, for example, by calculating the displacement between the center position of the alignment scale and the above position of the center of gravity. The movement direction and the movement distance of the fundus camera unit 2 can be determined by referring to a preset unit movement distances in the x-direction, y-direction, and z-direction (e.g., the result of prior measurement of how much the alignment indicator moves in which direction, when the fundus camera unit 2 is moved by how much in which direction). The main controller 211 generates signals according to the determined movement direction and movement distance, and transmits these signals to the movement mechanism 150. Thereby, the position of the optical system relative to the eye E to be examined is changed automatically.

### (S3: Set scan region)

The main controller 211 sets the configuration parameter for setting the scan region of the circle scan in the provisional measurement. For example, the main controller 211 sets the configuration parameter so that the site of interest in the eye E to be examined is positioned at the scan center position of the circle scan. In some embodiments, the main controller 211 sets the configuration parameter described above based on the operation content to the operation unit 240B.

In some embodiments, when the scan region for performing the circle scan is set, the main controller 211 sets a central region SAR1 including the optical axis of the optical system in the eye E to be examined, an intermediate region SAR2 around the central region SAR1, and a peripheral region SAR3 around the intermediate region SAR2, as shown in FIG. 9. The intermediate region SAR2 and the peripheral region SAR3 are an example of the "one or more peripheral regions" according to the embodiments.

In some embodiments, the configuration parameter described above includes parameter(s) for setting the central region SAR1, the intermediate region SAR2, and the peripheral region SAR3. In this case, the main controller 211 sets the central region SAR1, the intermediate region SAR2, and the peripheral region SAR3 based on the configuration parameter described above.

### (S4: Perform OCT scan)

Subsequently, the main controller 211 controls the optical scanner 42, the OCT unit 100, and the like to perform circle scan on each of a plurality of scan regions set in step S3. Specifically, the main controller 211 causes a circle scan to be performed in the central region SAR1, causes a circle scan to be performed in the intermediate region SAR2, and causes a circle scan to be performed in the peripheral region SAR3.

### (S5: Form OCT image)

Next, the main controller 211 controls the image forming unit 220 to sequentially form the OCT images based on the OCT data acquired by performing OCT scans on the plurality of scan regions sequentially performed in step S4.

### (S6: Calculate image quality evaluation value)

Subsequently, the main controller 211 controls the image quality evaluation value calculator 231A to calculate the image quality evaluation value for each of the plurality of OCT images formed for each scan region in step S5.

In some embodiments, the image quality evaluation value calculator 231A calculates the image quality evaluation value of a single image obtained by synthesizing a plurality of OCT images formed for each scan region in step S5.

### (S7: Search for optical condition of optical system that maximizes image quality evaluation value)

Subsequently, the main controller 211 searches for the optical condition(s) of the optical system for each scan region so that the image quality evaluation value calculated in step S6 becomes maximum, and identifies the optical condition(s) of the optical system that maximizes (maximize) the image quality evaluation value for each scan region.

In the present embodiments, examples of the optical condition(s) of the optical system include a focusing position of the measurement light LS, a polarization component of the measurement light LS, an aberration correction component, an optical path length difference between the measurement light LS and reference light LR, and a position of the optical system relative to the eye E to be examined.

The focusing position of the measurement light LS corresponds to a position on the optical axis of the OCT focusing lens 45. The polarization component of the measurement light LS corresponds to a polarization component of the light L0 changed by the polarization controller 103. The aberration correction component corresponds to a cylindrical power and a cylindrical axis angle changed by the VCC lens 47. The optical path length difference between the measurement light LS and the reference light LR corresponds to at least one of a position of the optical path length changing unit 41 (corner cube) on the optical path of the measurement light LS, or a position of the corner cube 114 on the optical path of the reference light LR. The position of the optical system relative to the eye E to be examined corresponds to a position in an optical direction of the optical system moved by the movement mechanism 150, and a position in a direction intersecting the optical axis of the optical system moved by the movement mechanism 150.

In some embodiments, in step S7, the main controller 211 searches for the optical condition(s) of the optical system so that the statistical value of the image quality evaluation values for each scan region calculated in step S6 becomes maximum, and identifies the optical condition(s) of the optical system that maximizes (maximize) the statistical value of the image quality evaluation values. In this case, example of the statistical value include an average value.

### (S8: Next?)

Subsequently, the main controller 211 determines whether or not the search for the optical condition(s) of the optical system should be continued. For example, the main controller 211 determines that the search for the optical condition(s) of the optical system should be continued when it is determined that the optical condition(s) of the optical system that maximizes the image quality evaluation value is (are) not identified for all of the plurality of scan regions. For example, the main controller 211 determines that the search for the optical condition(s) of the optical system should not be continued when it is determined that the optical condition(s) of the optical system that maximizes the image quality evaluation value is (are) identified for all of the plurality of scan regions.

When it is determined in step S8 that the search for the optical condition(s) of the optical system should be continued (S8: Y), the operation of the ophthalmic apparatus 1 proceeds to step S9. When it is determined in step S8 that the search for the optical condition(s) of the optical system should not be continued (S8: N), the operation of the ophthalmic apparatus 1 proceeds to step S10.

### (S9: Change optical condition of optical system)

When it is determined in step S8 that the search for the optical condition(s) of the optical system should be continued (S8: Y), the main controller 211 changes the optical condition(s) of the optical system. In this case, the main controller 211 changes the optical condition of the optical system by changing one of the parameters that define the optical condition(s) of the optical system by a predetermined step.

For example, the main controller 211 controls the OCT focusing driver 45A to change the optical condition(s) of the optical system by moving the position on the optical axis of the OCT focusing lens 45 by a predetermined step.

For example, the main controller 211 controls the polarization controller 103 to change the optical condition(s) of the optical system by changing the polarization component of the light L0 by a predetermined step.

For example, the main controller 211 controls the VCC driver 47A to change the optical condition(s) of the optical system by changing the cylindrical power of the VCC lens 47 or the cylindrical axis angle of the VCC lens 47 by a predetermined step.

For example, the main controller 211 controls the optical path length changing unit 41 or the reference driver 114A to change the optical condition(s) of the optical system by changing the position of the optical path length changing unit 41 (corner cube) on the optical path of the measurement light LS or the position of the corner cube 114 on the optical path of the reference light LR by a predetermined step.

For example, the main controller 211 controls the movement mechanism 150 to change the optical condition(s) of the optical system by changing the position in the optical axis direction of the optical system relative to the eye E to be examined or the position in the direction intersecting the optical axis direction of the optical system by a predetermined step.

Subsequent to step S9, the operation of the ophthalmic apparatus 1 proceeds to step S4.

### (S10: Store setting information on optical system)

When it is determined in step S8 that the search for the optical condition(s) of the optical system should not be continued (S8: N), the main controller 211 stores the setting information in the storage unit 212 for each scan region. Here, the setting information includes the configuration parameters for setting the optical condition(s) of the optical system, which are identified by repeatedly executing steps S4 through S9, The main controller 211 can store the setting information including the calculated image quality evaluation value in the storage unit 212 for each scan region.

### (S11: Display OCT image and image quality evaluation value)

Subsequently, the display controller 211A causes the OCT image formed in step S5 and the image quality evaluation value calculated in step S6 to be displayed on the display unit 240A.

For example, as shown in FIG. 10, the display controller 211A causes the OCT images formed for each scan region in step S5 to be displayed on the display unit 240A in association with the evaluation values calculated for each scan region in step S6. In FIG. 10, the OCT image IMG1 acquired by performing circle scan on the central region SAR1 is displayed in association with the image quality evaluation value VL1 calculated for the OCT image IMG1. Further, the OCT image IMG2 acquired by performing circle scan on the intermediate region SAR2 is displayed in association with the image quality evaluation value VL2 calculated for the OCT image IMG2. Furthermore, the OCT image IMG3 acquired by performing circle scan on the peripheral region SAR3 is displayed in association with the image quality evaluation value VL3 calculated for the OCT image IMG3.

Moreover, for example, as shown in FIG. 11, the display controller 211A causes the synthetic image (three-dimensional image, front image, en-face image) obtained by composing the plurality of OCT images formed for each scan region in step S5 to be displayed on the display unit 240A in association with the evaluation values calculated for the synthetic image in step S6. In FIG. 11, the en-face image IMG0 as the synthetic image is displayed in association with the image quality evaluation value VL0 calculated for the en-face image IMG0. Further, the OCT image IMG1 acquired by performing circle scan on the central region SAR1 is displayed in association with the image quality evaluation value VL1 calculated for the OCT image IMG1. Further, the OCT image IMG2 acquired by performing circle scan on the intermediate region SAR2 is displayed in association with the image quality evaluation value VL2 calculated for the OCT image IMG2. Furthermore, the OCT image IMG3 acquired by performing circle scan on the peripheral region SAR3 is displayed in association with the image quality evaluation value VL3 calculated for the OCT image IMG3.

This terminates the operation of the provisional measurement of the ophthalmic apparatus 1 (END).

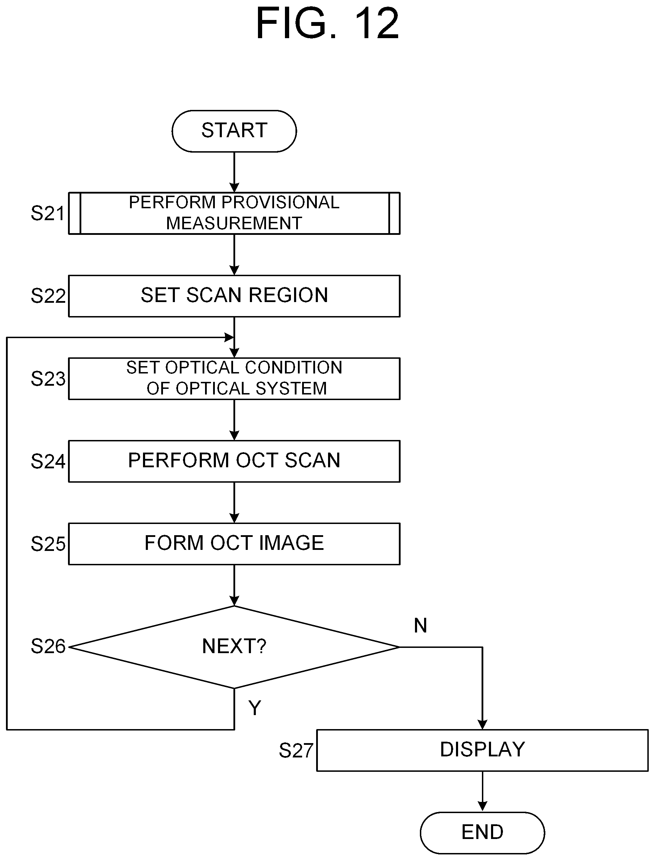

FIG. 12 shows a flowchart of a first operation example of the ophthalmic apparatus 1 according to the first embodiment. FIG. 12 mainly represents an example of the operation in the main measurement performed after the provisional measurement shown in FIG. 8. The storage unit 212 stores a computer program for realizing the processing shown in FIG. 12. The main controller 211 operates according to the computer program(s), and thereby the main controller 211 performs the processing shown in FIG. 12.

### (S21: Perform provisional measurement)

First, the main controller 211 performs provisional measurement. Step S21 is performed according to the flow shown in FIG. 8. Thereby, the setting information for setting the optical condition(s) of the optical system suitable for each scan region is identified, and the identified setting information is stored in the storage unit 212. In addition, the user can causes the main measurement following the provisional measurement to be performed while checking the image quality in the main measurement by referring to the OCT image displayed on the display unit 240A and the corresponding image quality evaluation value.

### (S22: Set scan region)

Next, the main controller 211 sets the scan mode (for example, ammonite scan) for the main measurement, and sets the scan region in the main measurement to the eye E to be examined. In the case that the scan mode in the main measurement is predetermined and the scan region in the main measurement is the same as the scan region in the provisional measurement, step S22 can be omitted.

### (S23: Set optical condition of optical system)

Subsequently, the main controller 211 identifies the scan position, where OCT scan is performed, based on the control content (control signal for controlling the deflection angle of the polarization plane, etc.) for the optical scanner 42 controlled in the main measurement, reads out the setting information from the storage unit 212 in accordance with the scan region including the identified scan position, and sets the optical condition(s) of the optical system based on the setting information that is read out.

### (S24: Perform OCT scan)

Next, the main controller 211 controls the optical scanner 42, the OCT unit 100, and the like to perform OCT scan in the main measurement on the set scan region.

### (S25: Form OCT image)

Subsequently, the main controller 211 controls the image forming unit 220 to form the OCT image based on the OCT data acquire in step S24.

### (S26: Next?)

Next, the main controller 211 determines whether or not the OCT scan in the next scan region should be continued. For example, the main controller 211 determines whether or not the OCT scan in the next scan region should be continued, by determining whether or not the preset scan region has been covered.

When it is determined in step S26 that the OCT scan in the next scan region should be continued (S26: Y), the operation of the ophthalmic apparatus 1 proceeds to step S23. When it is determined in step S26 that the OCT scan in the next scan region should not be continued (S26: N), the operation of the ophthalmic apparatus 1 proceeds to step S27.

### (S27: Display)

When it is determined in step S26 that the OCT scan in the next scan region should not be continued (S26: N), the display controller 211A causes the OCT image formed in step S25 to be displayed on the display unit 240A.

In some embodiments, the main controller 211 controls the data processor 230 to generate the synthetic image obtained by composing the plurality of OCT images sequentially formed by repeatedly executing steps S23 through S26. The display controller 211A can cause the generated synthetic image to be displayed on the display unit 240A.

This terminates the processing of the first operation example of the main measurement of the ophthalmic apparatus 1 (END).

As described above, according to the first operation example, OCT scan can be performed under the optimal optical conditions for each scan region. Thus, the scan region can be measured with high accuracy regardless of the cross-sectional shape of the eye E to be examined. As a result, even when imaging at wide angles, the images with suitable image quality can be acquired regardless of the scan region.

The operation of the ophthalmic apparatus 1 according to the first embodiment is not limited to the flow shown in FIG. 12. For example, the ophthalmic apparatus 1 according to the first embodiment may change the optical condition(s) of the optical system for each scan region so that the statistical value of the image quality evaluation values calculated for each scan region in the provisional measurement becomes maximum, and may perform OCT scan under the changed optical condition(s) for each scan region.

Alternatively, for example, the ophthalmic apparatus 1 according to the first embodiment may change the optical condition(s) of the optical system for each scan region so that the statistical value of the image quality evaluation values calculated for each scan region in the provisional measurement becomes maximum, and may perform wide-angle single OCT scan on all scan regions under the changed optical condition(s). Hereinafter, an operation example where this single OCT scan is performed will be described as a second operation example.

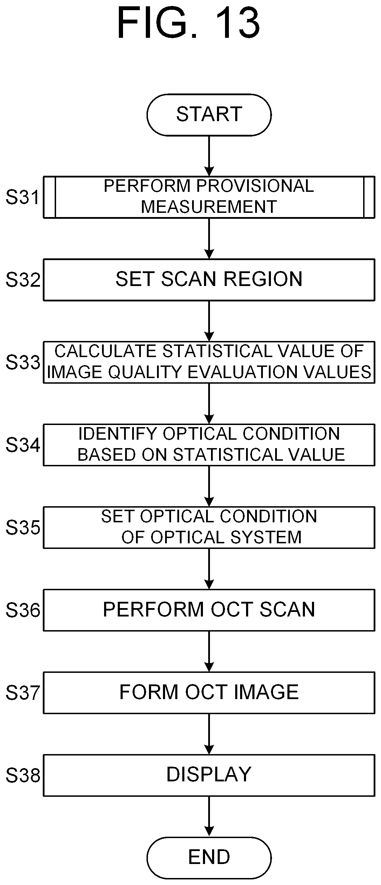

FIG. 13 shows a flowchart of the second operation example of the ophthalmic apparatus 1 according to the first embodiment. FIG. 13 mainly represents an example of the operation in the main measurement performed after the provisional measurement shown in FIG. 8. The storage unit 212 stores a computer program for realizing the processing shown in FIG. 13. The main controller 211 operates according to the computer program(s), and thereby the main controller 211 performs the processing shown in FIG. 13.

### (S31: Perform provisional measurement)

First, the main controller 211 performs provisional measurement, in the same manner as in step S21.

### (S32: Set scan region)

Next, the main controller 211 sets the scan mode (for example, ammonite scan) for the main measurement, and sets the scan region in the main measurement to the eye E to be examined, in the same manner as in step S22. In the case that the scan mode in the main measurement is predetermined and the scan region in the main measurement is the same as the scan region in the provisional measurement, step S32 can be omitted.

### (S33: Calculate statistical value of image quality evaluation values)

Subsequently, the main controller 211 controls the analyzer 231 (or image quality evaluation value calculator 231A) to calculate the statistical value of the image quality evaluation values calculated by the image quality evaluation value calculator 231A in the provisional measurement of each scan region, based on the scan region set in step S32, etc. Here, the statistical value may be an average value.

Step S33 may be performed in the provisional measurement in step S31.

### (S34: Identify optical condition based on statistical value)

Next, the main controller 211 identifies the optical condition(s) of the optical system based on the statistical value calculated in step S33. For example, the main controller 211 identifies the optical condition(s) of the optical system so that the statistical value (average value) of the image quality evaluation values corresponding to a plurality of scan regions becomes maximum.

### (S35: Set optical condition of optical system)

Next, the main controller 211 sets the optical condition(s) of the optical system to be the optical condition(s) identified in step S34.

### (S36: Perform OCT scan)

Next, the main controller 211 controls the optical scanner 42, the OCT unit 100, and the like to perform single OCT scan in the main measurement on the entire scan region, under the optical condition(s) set in step S35.

### (S37: Form OCT image)

Subsequently, the main controller 211 controls the image forming unit 220 to form the OCT image based on the OCT data acquire in step S36.

### (S38: Display)

Next, the display controller 211A causes the OCT image formed in step S37 to be displayed on the display unit 240A.

This terminates the processing of the second operation example of the main measurement of the ophthalmic apparatus 1 (END).

As described above, according to the second operation example, OCT scan be performed under a single optical condition optimal for the entire scan region. Thus, the scan region can be measured with high accuracy regardless of the cross-sectional shape of the eye E to be examined. As a result, even when imaging at wide angles, the images with suitable image quality can be acquired regardless of the scan region.

It should be noted that a case, where the focusing position of the measurement light LS used for OCT, the polarization component of the measurement light LS, the aberration correction component, the optical path length difference between the measurement light LS and the reference light LR, and the position of the optical system relative to the eye E to be examined are changed for each scan region, has been described in the first embodiment. However, the configuration according to the embodiments is not limited to this. In some embodiments, at least one of the focusing position of the measurement light LS used for OCT, the polarization component of the measurement light LS, the aberration correction component, the optical path length difference between the measurement light LS and the reference light LR, or the position of the optical system relative to the eye E to be examined is changed for each scan region as the optical condition(s) of the optical system. In other words, as the optical condition(s) of the optical system, the focusing position of the measurement light LS used for OCT, the polarization component of the measurement light LS, the aberration correction component, the optical path length difference between the measurement light LS and the reference light LR, or the position of the optical system relative to the eye E to be examined may be changed for each scan region.

### <Second embodiment>

In the first embodiment, a case where the VCC lens 47 is provided in the ophthalmic apparatus as the aberration correction device has been described. However, the configuration according to the embodiments is not limited to this. For example, the ophthalmic apparatus according to the embodiments may be configured to include a wavefront aberration correction optical system as the aberration correction device.

Hereinafter, the configuration according to the second embodiment will be described, focusing on the differences from the configuration according to the first embodiment.

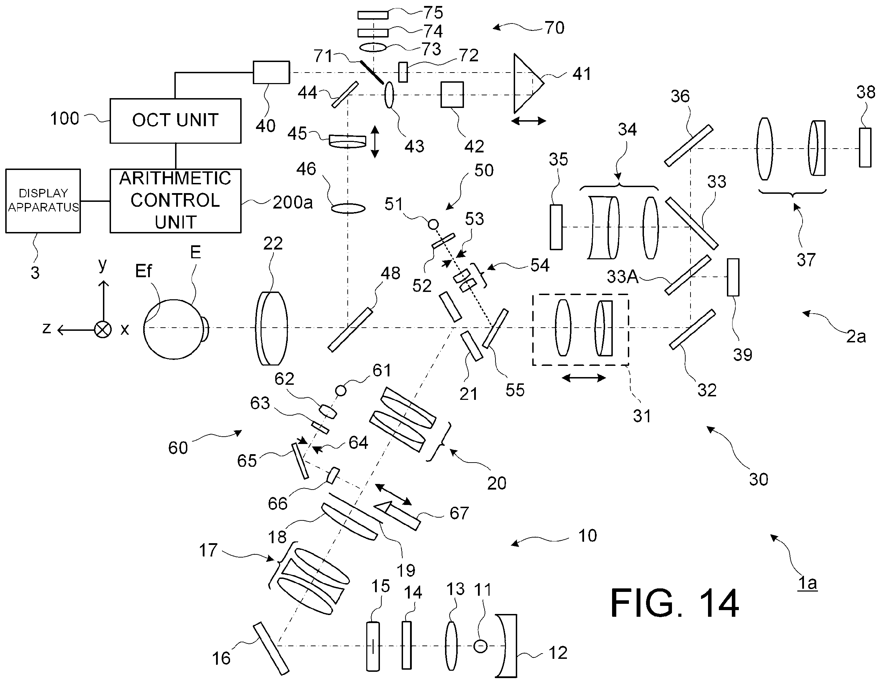

FIG. 14 shows an example of a configuration of an optical system of an ophthalmic apparatus 1a according to the second embodiment. In FIG. 14, the same parts as in FIG. 1 are shown by the same symbols and their descriptions will be omitted in an appropriate manner.

The configuration of the optical system in the ophthalmic apparatus 1a according to the second embodiment differs from that in the ophthalmic apparatus 1 according to the first embodiment in that the fundus camera unit 2a is provided instead of the fundus camera unit 2 and the arithmetic control unit 200a is provided instead of the arithmetic control unit 200.

The configuration of the fundus camera unit 2a differs from that of the fundus camera unit 2 in that a wavefront aberration correction optical system 70 is provided instead of the VCC lens 47. The configuration of the arithmetic control unit 200a differs from that of the arithmetic control unit 200 in that the arithmetic control unit 200a controls the wavefront aberration correction optical system 70 instead of the VCC lens 47.

The wavefront aberration correction optical system 70 includes a beam splitter 71, a transmissive adaptive optical element 72, a relay lens 73, a lens array 74, and a wavefront sensor 75.

In FIG. 14, the beam splitter 71 and the adaptive optical element 72 are arranged between the collimator lens unit 40 and the optical path length changing unit 41. The adaptive optical element 72 is arranged between the beam splitter 71 and the optical path length changing unit 41.

The beam splitter 71 splits the returning light of the measurement light LS into light guided to the collimator lens unit 40 side and light guided to the relay lens 73 side. The beam splitter 71 may be capable of changing a split ratio (branching ratio) of these lights. For example, when controlling the adaptive optical element 72 described below (for example, when performing the provisional measurement), the returning light of the measurement light LS is split into two lights with the split ratio of "50:50". And, when not controlling the adaptive optical element 72 (for example, when performing the main measurement), the returning light of the measurement light LS can be split into two lights with the split ratio of "90:10" so that the light quantity of the light guided to the collimator lens unit 40 side is increased.

The adaptive optical element 72 may be deformable phase plate (DPP), for example. The adaptive optical element 72 is an optical element that can locally change the phase of the wavefront of light passing through it.

In a reflection direction of the beam splitter 71, the relay lens 73 , the lens array 74, and the wavefront sensor 75 are arranged. Each of the adaptive optical element 72 and the lens array 74 is arranged at a position substantially conjugate optically to a pupil of the eye E to be examined (pupil conjugate position) or in the vicinity thereof.

The beam splitter 71 transmits the measurement light LS from the collimator lens unit 40 to guide the measurement light LS to the adaptive optical element 72, and guides the returning light of the measurement light LS from the eye E to be examined that has passed through the adaptive optical element 72 to the relay lens 73. The adaptive optical element 72 corrects at least the wavefront aberration of the returning light of the measurement light LS. The returning light of the measurement light LS that has been guided to the relay lens 73 passes through the relay lens 73, and is guided to the lens array 74.

The lens array 74 includes a plurality of lenses arranged in an array form, and generates a plurality of converging rays from the light passing through the relay lens 73. The plurality of converging rays generated by the lens array 74 is irradiated onto a sensor surface of the wavefront sensor 75. The wavefront sensor 75 may be a CMOS (complementary metal-oxide semiconductor) image sensor or a CCD (charge-coupled device) image sensor.

The wavefront of the light passing through the adaptive optical element 72 can be locally changed in accordance with detection results of the plurality of converging rays obtained by the wavefront sensor 75. Thereby, the adaptive optical element 72 can correct the wavefront aberration of the returning light of the measurement light LS from the eye E to be examined.

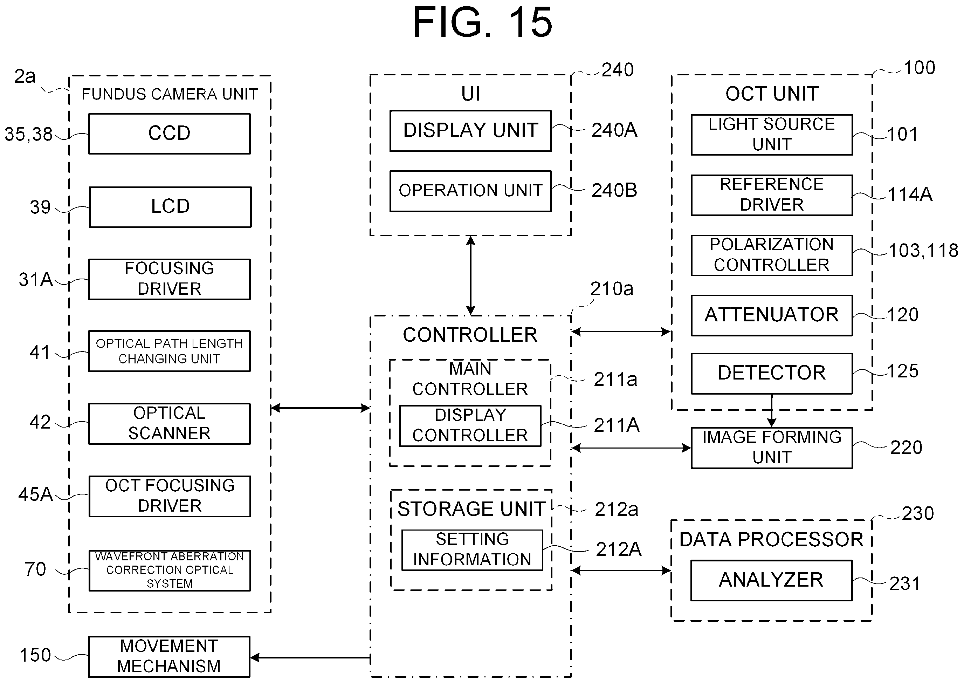

FIG. 15 shows a block diagram of an example of a configuration of a processing system of the ophthalmic apparatus 1a according to the second embodiment. In FIG. 15, the same parts as in FIG. 4 are shown by the same symbols and their descriptions will be omitted in an appropriate manner.

The configuration of the processing system of the ophthalmic apparatus 1a according to the second embodiment differs from the configuration of the processing system of the ophthalmic apparatus 1 according to the first embodiment mainly in that the wavefront aberration correction optical system 70 is provided instead of the VCC lens 47 and a controller 210a is provided instead of the controller 210.

The controller 210a includes a main controller 211a and a storage unit 212a. The controller 210a differs from the controller 210 in that the controller 210a controls the wavefront aberration correction optical system 70 instead of the VCC driver 47A.

In other words, according to the second embodiment, by controlling the wavefront aberration correction optical system 70 to change the aberration correction component, the optical condition(s) of the optical system can be changed.

The wavefront aberration correction optical system 70 is an example of the "aberration correction device" according to the embodiments. The optical system included in the OCT unit 100, the optical path length changing unit 41, the OCT focusing lens 45, and the wavefront aberration correction optical system 70 (interference optical system) are an example of the "optical system" according to the embodiments.

### <Third embodiment>

In the first embodiment or the second embodiment, the OCT image and the image quality evaluation value, which are acquired in the provisional measurement, are display unit 240A by associating them with each other. However, the configuration of the embodiments is not limited to this. For example, the OCT image acquired in the provisional measurement and information representing an evaluation metric of the image quality of the OCT image may be displayed on the display unit 240A. The information representing the evaluation metric of the image quality can be generated based on the image quality evaluation value, for example.

Hereinafter, the ophthalmic apparatus according to a third embodiment will be described, focusing on differences from the ophthalmic apparatus 1 according to the first embodiment.

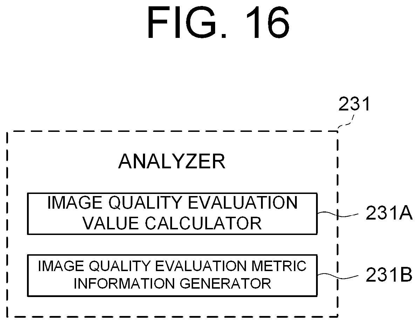

FIG. 16 shows a block diagram of an example of a configuration of the analyzer 231. In FIG. 16, the same parts as in FIG. 5 are shown by the same symbols and their descriptions will be omitted in an appropriate manner.

The configuration of the analyzer 231 according to the third embodiment shown in FIG. 16 differs from that of the analyzer 231 according to the first embodiment shown in FIG. 6 in that an image quality evaluation metric information generator 231B is added.

The image quality evaluation metric information generator 231B generates the information representing an evaluation metric of the image quality of the OCT image to be calculated for the image quality evaluation value. Examples of the information representing the evaluation metric of the image quality include information representing an evaluation of the image quality, information representing whether the image quality is good or poor (bad), and information representing the level of the image quality. Example of the information representing the evaluation metric include text representing the evaluation metric, an image representing the evaluation metric, and a color representing the evaluation metric.

For example, the image quality evaluation metric information generator 231B compares the image quality evaluation value generated by the image quality evaluation value calculator 231A with the image quality evaluation threshold value corresponding to a predetermined evaluation level, and generates the information representing the evaluation of the image quality corresponding to the comparison result.

For example, the image quality evaluation metric information generator 231B compares the image quality evaluation value generated by the image quality evaluation value calculator 231A with a predetermined image quality evaluation threshold value. As a result of the comparison, the image quality evaluation metric information generator 231B generates the information representing good or poor image quality. Here, the information representing good or poor image quality indicates that image quality is good when the image quality evaluation value is equal to or greater than the image quality evaluation threshold value. The information representing good or poor image quality indicates that image quality is poor when the image quality evaluation value is less than the image quality evaluation threshold value.

For example, the image quality evaluation metric information generator 231B compares the image quality evaluation value generated by the image quality evaluation value calculator 231A with one or more predetermined image quality evaluation threshold values different from each other. As a result of the comparison, the image quality evaluation metric information generator 231B generates the information representing the level of the image quality, based on the image quality evaluation threshold value with the highest image quality level among the image quality evaluation threshold values for which the image quality evaluation value is determined to be equal to or greater than the image quality evaluation thresholds.

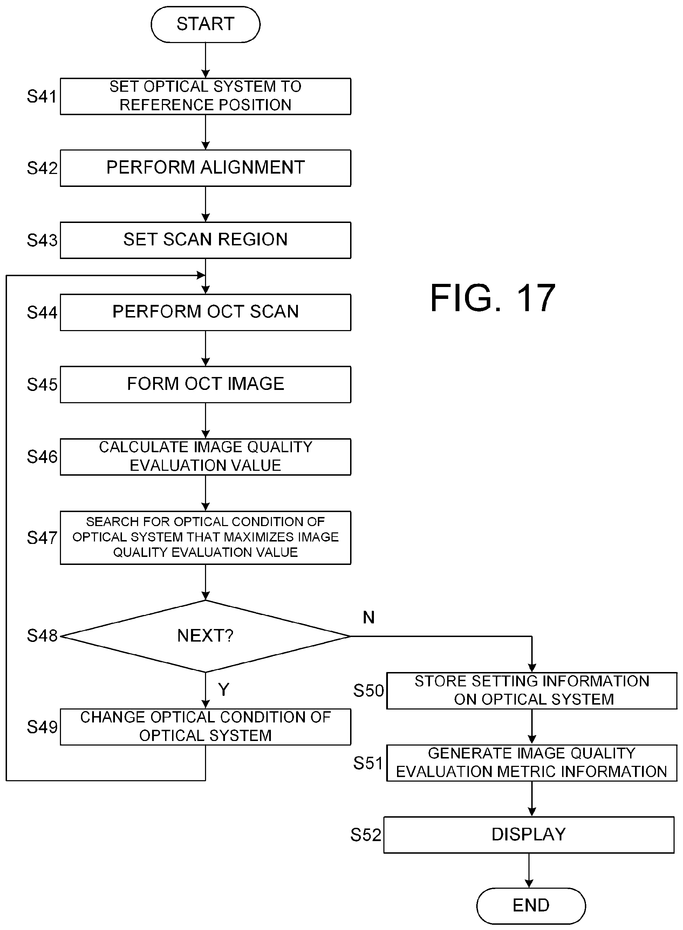

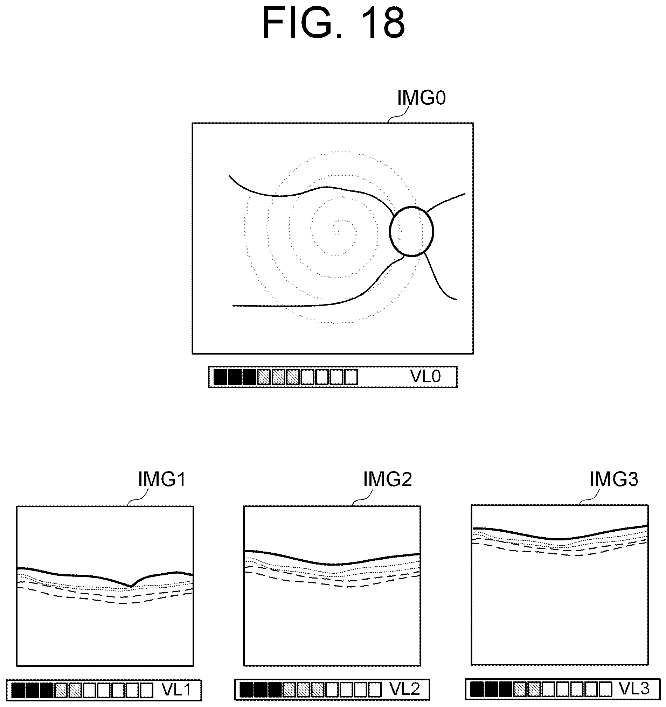

FIG. 17 and FIG. 18 show explanatory diagrams of the operation in the provisional measurement performed before the main measurement in the ophthalmic apparatus according to the third embodiment.

FIG. 17 represents a flowchart of the example of the operation in the provisional measurement of the ophthalmic apparatus according to the third embodiment. The storage unit 212 stores a computer program for realizing the processing shown in FIG. 17. The main controller 211 operates according to the computer program(s), and thereby the main controller 211 performs the processing shown in FIG. 17.

FIG. 18 schematically represents an example of displaying the image quality evaluation metric information in associated with the synthetic image of the plurality of OCT images and the OCT images of each scan region. Here, the OCT images are obtained by performing OCT scans in the provisional measurement which is performed in the ophthalmic apparatus according to the third embodiment. In FIG. 18, the same parts as in FIG. 11 are shown by the same symbols and their descriptions will be omitted in an appropriate manner.

### (S41: Set optical system to reference position)

First, the main controller 211 sets the optical conditions of the optical system constituting the ophthalmic apparatus 1 to a predetermined initial conditions, and sets the optical system to a reference position based on the set initial conditions, in the same manner as in step S1.

### (S42: Perform alignment)

Subsequently, the main controller 211 performs alignment adjustment of the optical system relative to the eye E to be examined, in the same manner as in step S2.

### (S43: Set scan region)

The main controller 211 sets the configuration parameter for setting the scan region of the circle scan in the provisional measurement, in the same manner as in step S3. For example, the main controller 211 sets the configuration parameter so that the site of interest in the eye E to be examined is positioned at the scan center position of the circle scan. In some embodiments, the main controller 211 sets the configuration parameter described above based on the operation content to the operation unit 240B.

### (S44: Perform OCT scan)

Subsequently, the main controller 211 controls the optical scanner 42, the OCT unit 100, and the like to perform circle scan on each of a plurality of scan regions set in step S43, in the same manner as in step S4. Specifically, the main controller 211 causes a circle scan to be performed in the central region SAR1, causes a circle scan to be performed in the intermediate region SAR2, and causes a circle scan to be performed in the peripheral region SAR3.

### (S45: Form OCT image)

Next, the main controller 211 controls the image forming unit 220 to sequentially form the OCT images based on the OCT data acquired by performing OCT scan on the plurality of scan regions sequentially performed in step S4, in the same manner as in step S5.

### (S46: Calculate image quality evaluation value)

Subsequently, the main controller 211 controls the image quality evaluation value calculator 231A to calculate the image quality evaluation value for each of the plurality of OCT images formed for each scan region in step S45, in the same manner as in step S6.

### (S47: Search for optical condition of optical system that maximizes image quality evaluation value)

Subsequently, the main controller 211 searches for the optical condition(s) of the optical system for each scan region so that the image quality evaluation value calculated in step S46 becomes maximum, and identifies the optical condition(s) of the optical system that maximizes (maximize) the image quality evaluation value for each scan region, in the same manner as in step S7.

In some embodiments, in step S47, the main controller 211 searches for the optical condition(s) of the optical system for each scan region so that the statistical value of the image quality evaluation values calculated in step S46 for each scan region becomes maximum, and identifies the optical condition(s) of the optical system that maximizes (maximize) the statistical value of the image quality evaluation values. In this case, example of the statistical value include an average value.

### (S48: Next?)

Subsequently, the main controller 211 determines whether or not the search for the optical condition(s) of the optical system should be continued, in the same manner as in step S8.

When it is determined in step S48 that the search for the optical condition(s) of the optical system should be continued (S48: Y), the operation of the ophthalmic apparatus proceeds to step S49. When it is determined in step S48 that the search for the optical condition(s) of the optical system should not be continued (S48: N), the operation of the ophthalmic apparatus proceeds to step S50.

### (S49: Change optical condition of optical system)

When it is determined in step S48 that the search for the optical condition(s) of the optical system should be continued (S48: Y), the main controller 211 changes the optical condition(s) of the optical system, in the same manner as in step S9. In this case, the main controller 211 changes the optical condition of the optical system by changing one of the parameters that defines the optical condition(s) of the optical system by a predetermined step.

Subsequent to step S49, the operation of the ophthalmic apparatus proceeds to step S44.

### (S50: Store setting information on optical system)

When it is determined in step S48 that the search for the optical condition(s) of the optical system should not be continued (S48: N), the main controller 211 stores the setting information for setting the optical condition(s) of the optical system, which is identified by repeatedly executing steps S44 through S49, in the storage unit 212 for each scan region, in the same manner as in step S10. The main controller 211 can store the setting information including the calculated image quality evaluation value in the storage unit 212 for each scan region.

### (S51: Generate image quality evaluation metric information)

Next, the main controller 211 controls the image quality evaluation metric information generator 231B to generate the image quality evaluation metric information based on the image quality evaluation value(s) calculated in step S46.

In some embodiments, the main controller 211 controls the image quality evaluation metric information generator 231B to generate the image quality evaluation metric information based on the image quality evaluation values calculated for the synthetic image obtained by composing the plurality of OCT images formed for each scan region in step S45.

### (S52: Display)

Subsequently, the display controller 211A causes the OCT image formed in step S45, the image quality evaluation value(s) calculated in step S46, and the image quality evaluation metric information generated in step S51 to be displayed on the display unit 240A.

For example, as shown in FIG. 18, the display controller 211A causes the OCT images formed for each scan region in step S45 to be displayed on the display unit 240A in association with the image quality evaluation value calculated for each scan region in step S46 and the image quality evaluation metric information calculated for each scan region in step S46.

In FIG. 18, the evaluation metric bar is displayed as the image quality evaluation metric information. The evaluation metric bar indicates that the higher the image quality level is, the greater the number of colored evaluation metric boxes is. For example, the evaluation metric bar with a predetermined number (for example, 3) that indicates good image quality is colored red, and when the image quality is better than the number, the evaluation metric bar is colored green in accordance with the image quality level. Thereby, the image quality level of the OCT image can be judged easily.

It should be noted that, in FIG. 18, the OCT image IMG1 acquired by performing circle scan on the central region SAR1 is displayed in association with the image quality evaluation value VL1 calculated for the OCT image IMG1 and the evaluation metric bar colored in accordance with the image quality evaluation value VL1. Further, the OCT image IMG2 acquired by performing circle scan on the intermediate region SAR2 is displayed in association with the image quality evaluation value VL2 calculated for the OCT image IMG2 and the evaluation metric bar colored in accordance with the image quality evaluation value VL2. Furthermore, the OCT image IMG3 acquired by performing circle scan on the peripheral region SAR3 is displayed in association with the image quality evaluation value VL3 calculated for the OCT image IMG3 and the evaluation metric bar colored in accordance with the image quality evaluation value VL2.

Moreover, for example, as shown in FIG. 18, the display controller 211A causes the synthetic image (three-dimensional image, front image, en-face image) obtained by composing the plurality of OCT images formed for each scan region in step S45 to be displayed on the display unit 240A in association with the image quality evaluation value VL0 calculated for the synthetic image and the image quality evaluation metric information generated based on the image quality evaluation value VL0. In FIG. 18, the en-face image IMG0 as the synthetic image is displayed in association with the image quality evaluation value VL0 calculated for the en-face image IMG0 and the evaluation metric bar colored in accordance with the image quality evaluation value VL0.

This terminates the operation in the provisional measurement of the ophthalmic apparatus according to the third embodiment (END).

### [Actions]

The OCT apparatus, the method of controlling the OCT apparatus, and the program according to the embodiments will be explained.

The first aspect of the embodiments is an OCT apparatus (ophthalmic apparatus 1, 1a) including an optical system (optical system included in the OCT unit 100, optical path length changing unit 41, OCT focusing lens 45, VCC lens 47 (or wavefront aberration correction optical system 70), interference optical system), a storage unit (212, 212a), and a controller (210, 210a, main controller 211, main controller 211a). The optical system is configured to allow an optical condition (focusing position of measurement light LS, polarization component of measurement light LS, aberration correction component, optical path length difference between measurement light LS and reference light LR, or position of the optical system relative to eye E to be examined) to be changed. The optical system is configured to acquire OCT data of an object to be measured (eye E to be examined) by scanning the object to be measured with measurement light (LS). The storage unit stores in advance setting information (212A) for each scan region in the object to be measured, the setting information being information for setting an optical condition of the optical system so that image quality of an image formed based on the OCT data becomes equal to or greater than a predetermined image quality level. The controller is configured to change an optical condition of the optical system based on the setting information to perform OCT on the object to be measured, in accordance with the scan region of the measurement light in the object to be measured.

According to such an aspect, the setting information for setting the optical conditions of the optical system is stored in advance for each scan region of the measurement light, and OCT is performed on the object to be measured by changing the optical condition(s) of the optical system based on the setting information in accordance with the scan region. Thereby, the image of the object to be measured can be acquired with good image quality regardless of the cross-sectional shape of the object to be measured, and the measurement time (imaging time) can be shortened.

In the second aspect of the embodiments, in the first embodiment, the scan region includes a central region (SAR1) including an optical axis of the optical system in the object to be measured, and one or more peripheral regions (intermediate region SAR2, peripheral region SAR3) around the central region.

According to such an aspect, the setting information for setting the optical conditions of the optical system is stored in advance for each of the central region and the one or more peripheral regions, and OCT is performed on the object to be measured by changing the optical condition(s) of the optical system based on the setting information in accordance with the scan region. Thereby, the image of the object to be measured can be acquired with good image quality for the central region and the one or more peripheral regions regardless of the cross-sectional shape of the object to be measured, and the measurement time (imaging time) can be shortened.

In the third aspect of the embodiments, in the first aspect or the second aspect, the optical system includes an interference optical system (optical system included in the OCT unit 100, optical path length changing unit 41, OCT focusing lens 45, VCC lens 47 (or wavefront aberration correction optical system 70)), an optical path length changing member (optical path length changing unit 41, or corner cube 114 and reference driver 114A). The interference optical system is configured to split light (L0) from a light source (light source unit 101) into the measurement light (LS) and reference light (LR), to project the measurement light having traveled through a measurement optical path, on which one or more optical elements (OCT focusing lens 45, corner cube 114, optical path length changing unit 41, polarization controller 103) are arranged, onto the object to be measured, and to detect interference light (LC) between returning light from the object to be measured and the reference light having traveled through a reference optical path. The optical path length difference changing member is configured to change a difference between an optical path length of the measurement light and an optical path length of the reference light. The controller is configured to change the optical condition of the optical system by controlling at least one of the one or more optical elements or the optical path length difference changing member based on the setting information, and to perform OCT on the object to be measured.

According to such an aspect, by controlling at least one of the one or more optical elements or optical path length difference changing member based on the setting information for each scan region, the optical condition of the optical system is changed to perform OCT on the object to be measured in accordance with the scan region. Thereby, the image of the object to be measured can be acquired with good image quality regardless of the cross-sectional shape of the object to be measured, and the measurement time (imaging time) can be shortened.

In the fourth aspect of the embodiments, in the third aspect, the one or more optical elements includes a focusing lens (OCT focusing lens 45) or an aberration correction device (VCC lens 47 or wavefront aberration correction optical system 70).

According to such an aspect, by controlling at least one of the one or more optical elements including the focusing lens or aberration correction device, or optical path length difference changing member based on the setting information for each scan region, the optical condition of the optical system is changed to perform OCT on the object to be measured in accordance with the scan region. Thereby, the image of the object to be measured can be acquired with good image quality regardless of the cross-sectional shape of the object to be measured, and the measurement time (imaging time) can be shortened.

The fifth aspect of the embodiments, in the first aspect or the second aspect, further includes a movement mechanism (150) configured to relatively move the optical system relative to the object to be measured. The controller is configured to change an optical condition of the optical system by controlling the movement mechanism based on the setting information, and to perform OCT on the object to be measured.

According to such an aspect, the setting information for setting the optical conditions of the optical system is stored in advance for each scan region of the measurement light, and the optical condition of the optical system is changed to perform OCT on the object to be measured by relatively moving the optical system relative to the object to be measured based on the setting information, in accordance with the scan region. Thereby, the image of the object to be measured can be acquired with good image quality regardless of the cross-sectional shape of the object to be measured, and the measurement time (imaging time) can be shortened.

The sixth aspect of the embodiments, in the first aspect or the second aspect, further includes an image forming unit (220), and an image quality evaluation value calculator (231A). The image forming unit is configured to form an image of the object to be measured for each scan region based on the OCT data. The image quality evaluation value calculator is configured to calculate an evaluation value of image quality (image quality evaluation value) of the image of the object to be measured. The controller is configured to identify an optical condition of the optical system to maximize image quality of the image based on the evaluation value, for each scan region.

According to such an aspect, the optical condition of the optical system is identified based on the evaluation value of the image quality of the image for each scan region. Thereby, the image of the object to be measured can be acquired with good image quality regardless of the cross-sectional shape of the object to be measured, and the measurement time (imaging time) can be shortened.

In the seventh aspect of the embodiments, in the sixth aspect, the controller is configured to cause the evaluation value of image quality of the image of the object to be measured to be displayed on a display means (display unit 240A, display apparatus 3), for each scan region.

According to such an aspect, the evaluation value of the image quality displayed on the display means can be referred to for each scan region. Thereby, the main measurement of OCT can be performed while checking the image quality that changes in accordance with the cross-sectional shape of the object to be measured. As a result, the opportunity for re-measurement (re-imaging) is reduced and the measurement time (imaging time) can be shortened.

In the eighth aspect of the embodiments, in the seventh aspect, the controller is configured to cause the image in the scan region formed by the image forming unit to be displayed on the display means in association with the evaluation value.

According to such an aspect, the image in the scan region formed by the image forming unit is displayed on display means in association with the evaluation value. Thereby, the image quality of the image can be easily checked.

In the ninth aspect of the embodiments, in the sixth aspect, the controller is configured to cause evaluation metric information representing an evaluation metric of image quality (image quality evaluation metric information) of the image of the object to be measured to be displayed on a display means (display unit 240A, display apparatus 3), for each scan region.

According to such an aspect, the image quality of the image of the object to be measured can be evaluated based on the evaluation metric information. Thereby, the image quality of the image can be easily checked.

In the tenth aspect of the embodiments, in the ninth embodiment, the controller is configured to cause the image in the scan region formed by the image forming unit to be displayed on the display means in association with the evaluation metric information.

According to such an aspect, the image in the scan region formed by the image forming unit is displayed on display means in association with the evaluation metric information. Thereby, the image quality of the image can be checked more easily.

In the eleventh aspect of the embodiments, in the sixth aspect, the controller is configured to change an optical condition of the optical system based on a statistical value (maximum value, minimum value, median, average value, mode, range, variance, standard deviation, weighted average value where a weighting factor is larger the closer to the site of interest (optical axis of the optical system), or a value of a predetermined evaluation equation using any of the statistical values described above) of evaluation values of image quality of images in a plurality of scan regions, and to perform OCT on the object to be measured.

According to such an aspect, the statistical value of the evaluation value of the image quality displayed on the display means can be referred to for each scan region. Thereby, the main measurement of OCT can be performed while checking the image quality that changes in accordance with the cross-sectional shape of the object to be measured. As a result, the opportunity for re-measurement (re-imaging) is reduced and the measurement time (imaging time) can be shortened.

The twelfth aspect of the embodiments is a method of controlling an OCT apparatus including an optical system (optical system included in the OCT unit 100, optical path length changing unit 41, OCT focusing lens 45, and VCC lens 47 (or wavefront aberration correction optical system 70), interference optical system). The optical system is configured to acquire OCT data of an object to be measured by scanning the object to be measured with measurement light (LS). The optical system is configured to allow an optical condition (focusing position of measurement light LS, polarization component of measurement light LS, aberration correction component, optical path length difference between measurement light LS and reference light LR, or position of the optical system relative to eye E to be examined) to be changed. The method of controlling the OCT apparatus includes a control step of changing an optical condition of the optical system based on setting information, the setting information being information for setting an optical condition of the optical system so that image quality of an image formed based on the OCT data becomes equal to or greater than a predetermined image quality level, and of performing OCT on the object to be measured, for each scan region in the object to be measured, in accordance with the scan region of the measurement light in the object to be measured.

According to such an aspect, the setting information for setting the optical conditions of the optical system is stored in advance for each scan region of the measurement light, and OCT is performed on the object to be measured by changing the optical condition(s) of the optical system based on the setting information in accordance with the scan region. Thereby, the image of the object to be measured can be acquired with good image quality regardless of the cross-sectional shape of the object to be measured, and the measurement time (imaging time) can be shortened.

In the thirteenth aspect of the embodiments, in the twelfth aspect, the scan region includes a central region (SAR1) including an optical axis of the optical system in the object to be measured, and one or more peripheral regions (intermediate region SAR2, peripheral region SAR3) around the central region.

According to such an aspect, the setting information for setting the optical conditions of the optical system is stored in advance for each of the central region and the one or more peripheral regions, and OCT is performed on the object to be measured by changing the optical condition(s) of the optical system based on the setting information in accordance with the scan region. Thereby, the image of the object to be measured can be acquired with good image quality in the central region and the one or more peripheral regions regardless of the cross-sectional shape of the object to be measured, and the measurement time (imaging time) can be shortened.

In the fourteenth aspect of the embodiments, in the twelfth aspect or the thirteenth aspect, the optical system further includes an interference optical system (optical system included in the OCT unit 100, optical path length changing unit 41, OCT focusing lens 45, VCC lens 47 (or wavefront aberration correction optical system 70)), an optical path length changing member (optical path length changing unit 41, or corner cube 114 and reference driver 114A). The interference optical system is configured to split light (L0) from a light source (light source unit 101) into the measurement light (LS) and reference light (LR), to project the measurement light having traveled through a measurement optical path, on which one or more optical elements (OCT focusing lens 45, corner cube 114, optical path length changing unit 41, polarization controller 103) are arranged, onto the object to be measured, and to detect interference light (LC) between returning light from the object to be measured and the reference light having traveled through a reference optical path. The optical path length difference changing member is configured to change a difference between an optical path length of the measurement light and an optical path length of the reference light. The control step is performed to change an optical condition of the optical system by controlling at least one of the one or more optical elements or the optical path length difference changing member based on the setting information, and to perform OCT on the object to be measured.

According to such an aspect, by controlling at least one of the one or more optical elements or optical path length difference changing member based on the setting information for each scan region, the optical condition of the optical system is changed to perform OCT on the object to be measured in accordance with the scan region. Thereby, the image of the object to be measured can be acquired with good image quality regardless of the cross-sectional shape of the object to be measured, and the measurement time (imaging time) can be shortened.

In the fifteenth aspect of the embodiments, in the fourteenth aspect, the one or more optical elements includes a focusing lens (OCT focusing lens 45) or an aberration correction device (VCC lens 47 or wavefront aberration correction optical system 70).

According to such an aspect, by controlling at least one of the one or more optical elements including the focusing lens or aberration correction device, or optical path length difference changing member based on the setting information for each scan region, the optical condition of the optical system is changed to perform OCT on the object to be measured in accordance with the scan region. Thereby, the image of the object to be measured can be acquired with good image quality regardless of the cross-sectional shape of the object to be measured, and the measurement time (imaging time) can be shortened.

In the sixteenth aspect of the embodiments, in the twelfth aspect or the thirteenth aspect, the OCT apparatus includes a movement mechanism (150) configured to relatively move the optical system relative to the object to be measured. The control step is performed to change an optical condition of the optical system by controlling the movement mechanism based on the setting information, and to perform OCT on the object to be measured.

According to such an aspect, the setting information for setting the optical conditions of the optical system is stored in advance for each scan region, and the optical condition of the optical system is changed to perform OCT on the object to be measured by relatively moving the optical system relative to the object to be measured based on the setting information, in accordance with the scan region. Thereby, the image of the object to be measured can be acquired with good image quality regardless of the cross-sectional shape of the object to be measured, and the measurement time (imaging time) can be shortened.

The seventeenth aspect of the embodiments, in the twelfth aspect or the thirteenth aspect, further includes an image forming step and an image quality evaluation value calculation step. The image forming step is performed to form an image of the object to be measured for each scan region based on the OCT data. The image quality evaluation value calculation step is performed to calculate an evaluation value of image quality (image quality evaluation value) of the image of the object to be measured. The control step is performed to identify an optical condition of the optical system to maximize image quality of the image based on the evaluation value, for each scan region.

According to such an aspect, the optical condition of the optical system is identified based on the evaluation value of the image quality of the image for each scan region. Thereby, the image of the object to be measured can be acquired with good image quality regardless of the cross-sectional shape of the object to be measured, and the measurement time (imaging time) can be shortened.

The eighteenth aspect of the embodiments, in the seventeenth aspect, further includes a display control step of causing the evaluation value of image quality of the image of the object to be measured to be displayed on a display means (display unit 240A, display apparatus 3), for each scan region.

According to such an aspect, the evaluation value of the image quality displayed on the display means can be referred to for each scan region. Thereby, the main measurement of OCT can be performed while checking the image quality that changes in accordance with the cross-sectional shape of the object to be measured. As a result, the opportunity for re-measurement (re-imaging) is reduced and the measurement time (imaging time) can be shortened.

In the nineteenth aspect of the embodiments, in the eighteenth aspect, the display control step is performed to cause the image in the scan region formed in the image forming step to be displayed on the display means in association with the evaluation value.

According to such an aspect, the image in the scan region formed in the image forming step is displayed on display means in association with the evaluation value. Thereby, the image quality of the image can be easily checked.

The twentieth aspect of the embodiments, in the seventeenth aspect, further includes a display control step of causing the evaluation metric information of image quality of the image (image quality evaluation metric information) of the object to be measured to be displayed on a display means (display unit 240A, display apparatus 3), for each scan region.

According to such an aspect, the image quality of the image of the object to be measured can be evaluated based on the evaluation metric information. Thereby, the image quality of the image can be easily checked.

In the twenty-first aspect of the embodiments, in the twentieth aspect, the display control step is performed to cause the image in the scan region formed in the image forming step to be displayed on the display means in association with the evaluation metric information.

According to such an aspect, the image in the scan region formed by the image forming unit is displayed on display means in association with the evaluation metric information. Thereby, the image quality of the image can be checked more easily.

In the twenty-second aspect of the embodiments, in the seventeenth aspect, the control step is performed to change an optical condition of the optical system based on a statistical value (maximum value, minimum value, median, average value, mode, range, variance, standard deviation, weighted average value where a weighting factor is larger the closer to the site of interest (optical axis of the optical system), or a value of a predetermined evaluation equation using any of the statistical values described above) of evaluation values of image quality of images in a plurality of scan regions, and to perform optical coherence tomography on the object to be measured.

According to such an aspect, the statistical value of the evaluation value of the image quality displayed on the display means can be referred to for each scan region. Thereby, the main measurement of OCT can be performed while checking the image quality that changes in accordance with the cross-sectional shape of the object to be measured. As a result, the opportunity for re-measurement (re-imaging) is reduced and the measurement time (imaging time) can be shortened.

The twenty-third aspect is a program of causing a computer to execute each step of the method of controlling the OCT apparatus in any one of the twelfth aspect to the twenty-second aspect.

According to such an aspect, the setting information for setting the optical conditions of the optical system is stored in advance for each scan region of the measurement light, and OCT is performed on the object to be measured by changing the optical condition(s) of the optical system based on the setting information in accordance with the scan region. Thereby, the image of the object to be measured can be acquired with good image quality regardless of the cross-sectional shape of the object to be measured, and the measurement time (imaging time) can be shortened.

The configuration described above is only an example for suitably implementing the present invention. Therefore, any modification (omission, substitution, addition, etc.) within the scope of the gist of the present invention can be appropriately applied. The configuration to be employed is selected according to the purpose, for example. In addition, depending on the configuration to be applied, it is possible to obtain the actions and effects obvious to those skilled in the art and the actions and effects described in this specification.

### [Explanation of symbols]

- 1, 1a: Ophthalmic apparatus
- 3: Display apparatus
- 41: Optical path length changing unit
- 45: OCT focusing lens
- 47: VCC lens
- 47A: VCC driver
- 70: Wavefront aberration correction optical system
- 100: OCT unit
- 103, 118: Polarization controller
- 114: Corner cube
- 114A: Reference driver
- 200 , 200a: Arithmetic control unit
- 210, 210a: Controller
- 211, 211a: Main controller
- 211A: Display controller
- 212, 212a: Storage unit
- 212A: Setting information
- 220: Image forming unit
- 230: Data processor
- 231: Analyzer
- 231A: Image quality evaluation value calculator
- 231B: Image quality evaluation metric information generator
- 240A: Display unit
- E: Eye to be examined
- SAR1: Central region
- SAR2: Intermediate region
- SAR3: Peripheral region
- LC: Interference light
- LR: Reference light
- LS: Measurement light

## Claims

1. An optical coherence tomography apparatus, comprising:
an optical system configured to allow an optical condition to be changed, and to acquire optical coherence tomography data of an object to be measured by scanning the object to be measured with measurement light;
a storage unit that stores in advance setting information for each scan region in the object to be measured, the setting information being information for setting an optical condition of the optical system so that image quality of an image formed based on the optical coherence tomography data becomes equal to or greater than a predetermined image quality level; and
a controller configured to change an optical condition of the optical system based on the setting information to perform optical coherence tomography on the object to be measured, in accordance with the scan region of the measurement light in the object to be measured.

2. The optical coherence tomography apparatus of claim 1, wherein
the scan region includes a central region including an optical axis of the optical system in the object to be measured, and one or more peripheral regions around the central region.

3. The optical coherence tomography apparatus of claim 1 or 2, wherein
the optical system includes:
an interference optical system configured to split light from a light source into the measurement light and reference light, to project the measurement light having traveled through a measurement optical path, on which one or more optical elements are arranged, onto the object to be measured, and to detect interference light between returning light from the object to be measured and the reference light having traveled through a reference optical path; and
an optical path length difference changing member configured to change a difference between an optical path length of the measurement light and an optical path length of the reference light, wherein
the controller is configured to change the optical condition of the optical system by controlling at least one of the one or more optical elements or the optical path length difference changing member based on the setting information, and to perform optical coherence tomography on the object to be measured.

4. The optical coherence tomography apparatus of claim 3, wherein
the one or more optical elements includes a focusing lens or an aberration correction device.

5. The optical coherence tomography apparatus of claim 1 or 2, further comprising
a movement mechanism configured to relatively move the optical system relative to the object to be measured, wherein
the controller is configured to change an optical condition of the optical system by controlling the movement mechanism based on the setting information, and to perform optical coherence tomography on the object to be measured.

6. The optical coherence tomography apparatus of claim 1 or 2, further comprising
an image forming unit configured to form an image of the object to be measured for each scan region based on the optical coherence tomography data; and
an image quality evaluation value calculator configured to calculate an evaluation value of image quality of the image of the object to be measured, wherein
the controller is configured to identify an optical condition of the optical system to maximize image quality of the image based on the evaluation value, for each scan region.

7. The optical coherence tomography apparatus of claim 6, wherein
the controller is configured to cause the evaluation value of image quality of the image of the object to be measured to be displayed on a display means, for each scan region.

8. The optical coherence tomography apparatus of claim 7, wherein
the controller is configured to cause the image in the scan region formed by the image forming unit to be displayed on the display means in association with the evaluation value.

9. The optical coherence tomography apparatus of claim 6, wherein
the controller is configured to cause evaluation metric information representing an evaluation metric of image quality of the image of the object to be measured to be displayed on a display means, for each scan region.

10. The optical coherence tomography apparatus of claim 9, wherein
the controller is configured to cause the image in the scan region formed by the image forming unit to be displayed on the display means in association with the evaluation metric information.

11. The optical coherence tomography apparatus of claim 6, wherein
the controller is configured to change an optical condition of the optical system based on a statistical value of evaluation values of image quality of images in a plurality of scan regions, and to perform optical coherence tomography on the object to be measured.

12. A method of controlling an optical coherence tomography apparatus including an optical system configured to allow an optical condition to be changed, and to acquire optical coherence tomography data of an object to be measured by scanning the object to be measured with measurement light, the method comprising
a control step of changing an optical condition of the optical system based on setting information, the setting information being information for setting an optical condition of the optical system so that image quality of an image formed based on the optical coherence tomography data becomes equal to or greater than a predetermined image quality level, and of performing optical coherence tomography on the object to be measured, for each scan region in the object to be measured, in accordance with the scan region of the measurement light in the object to be measured.

13. The method of controlling the optical coherence tomography apparatus of claim 12, wherein
the scan region includes a central region including an optical axis of the optical system in the object to be measured, and one or more peripheral regions around the central region.

14. The method of controlling the optical coherence tomography apparatus of claim 12 or 13, wherein
the optical system further includes:
an interference optical system configured to split light from a light source into the measurement light and reference light, to project the measurement light having traveled through a measurement optical path, on which one or more optical elements are arranged, onto the object to be measured, and to detect interference light between returning light from the object to be measured and the reference light having traveled through a reference optical path; and
an optical path length difference changing member configured to change a difference between an optical path length of the measurement light and an optical path length of the reference light, wherein
the control step is performed to change an optical condition of the optical system by controlling at least one of the one or more optical elements or the optical path length difference changing member based on the setting information, and to perform optical coherence tomography on the object to be measured.

15. The method of controlling the optical coherence tomography apparatus of claim 14, wherein
the one or more optical elements includes a focusing lens or an aberration correction device.

16. The method of controlling the optical coherence tomography apparatus of claim 12 or 13, wherein
the optical coherence tomography apparatus includes a movement mechanism configured to relatively move the optical system relative to the object to be measured, and
the control step is performed to change an optical condition of the optical system by controlling the movement mechanism based on the setting information, and to perform optical coherence tomography on the object to be measured.

17. The method of controlling the optical coherence tomography apparatus of claim 12 or 13, further comprising
an image forming step of forming an image of the object to be measured for each scan region based on the optical coherence tomography data; and
an image quality evaluation value calculation step of calculating an evaluation value of image quality of the image of the object to be measured, wherein
the control step is performed to identify an optical condition of the optical system to maximize image quality of the image based on the evaluation value, for each scan region.

18. The method of controlling the optical coherence tomography apparatus of claim 17, further comprising
a display control step of causing the evaluation value of image quality of the image of the object to be measured to be displayed on a display means, for each scan region.

19. The method of controlling the optical coherence tomography apparatus of claim 18, wherein
the display control step is performed to cause the image in the scan region formed in the image forming step to be displayed on the display means in association with the evaluation value.

20. The method of controlling the optical coherence tomography apparatus of claim 17, further comprising
a display control step of causing the evaluation metric information representing an evaluation metric of image quality of the image of the object to be measured to be displayed on a display means, for each scan region.

21. The method of controlling the optical coherence tomography apparatus of claim 20, wherein
the display control step is performed to cause the image in the scan region formed in the image forming step to be displayed on the display means in association with the evaluation metric information.

22. The method of controlling the optical coherence tomography apparatus of claim 17, wherein
the control step is performed to change an optical condition of the optical system based on a statistical value of evaluation values of image quality of images in a plurality of scan regions, and to perform optical coherence tomography on the object to be measured.

23. A program of causing a computer to execute each step of the method of controlling the optical coherence tomography apparatus of claim 12 or 13.
